(19) Europäisches Patentamt / European Patent Office / Office européen des brevets

(11) EP 3 275 884 B1

(12) **EUROPEAN PATENT SPECIFICATION**

(45) Date of publication and mention of the grant of the patent:
**08.05.2019 Bulletin 2019/19**

(21) Application number: **16768792.0**

(22) Date of filing: **23.03.2016**

(51) Int Cl.:
***C07D 495/04*** (2006.01) ***H01L 21/336*** (2006.01)
***H01L 29/786*** (2006.01) ***H01L 51/05*** (2006.01)
***H01L 51/30*** (2006.01) ***H01L 51/40*** (2006.01)
***H01L 51/46*** (2006.01)

(86) International application number:
**PCT/JP2016/059113**

(87) International publication number:
**WO 2016/152889 (29.09.2016 Gazette 2016/39)**

(54) **ORGANIC COMPOUND, ORGANIC SEMICONDUCTOR MATERIAL, ORGANIC THIN-FILM AND METHOD FOR PRODUCING SAME, ORGANIC SEMICONDUCTOR COMPOSITION, AND ORGANIC SEMICONDUCTOR DEVICE**

ORGANISCHE VERBINDUNG, ORGANISCHES HALBLEITERMATERIAL, ORGANISCHE DÜNNSCHICHT UND VERFAHREN ZUR HERSTELLUNG DAVON, ORGANISCHE HALBLEITERZUSAMMENSETZUNG UND ORGANISCHES HALBLEITERBAUELEMENT

COMPOSÉ ORGANIQUE, MATÉRIAU ORGANIQUE SEMI-CONDUCTEUR, FILM ORGANIQUE MINCE ET SON PROCÉDÉ DE PRODUCTION, COMPOSITION ORGANIQUE SEMI-CONDUCTRICE ET DISPOSITIF ORGANIQUE SEMI-CONDUCTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2015 JP 2015059624**

(43) Date of publication of application:
**31.01.2018 Bulletin 2018/05**

(73) Proprietor: **Nippon Kayaku Kabushiki Kaisha**
**Tokyo 100-0005 (JP)**

(72) Inventors:
- **HAMADA, Masahiro**
  **Tokyo 115-8588 (JP)**
- **SHINAMURA, Shoji**
  **Tokyo 115-8588 (JP)**
- **SADAMITSU, Yuichi**
  **Tokyo 115-8588 (JP)**

(74) Representative: **Gille Hrabal**
**Brucknerstrasse 20**
**40593 Düsseldorf (DE)**

(56) References cited:
**JP-A- 2010 034 450** **JP-A- 2011 258 900**
**JP-A- 2014 531 435**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 275 884 B1

## Description

### Technical Field

The present invention relates to an organic compound (a condensed polycyclic aromatic compound), an organic semiconductor material containing the same, an organic thin film and a method for producing the same, an organic semiconductor composition, and an organic semiconductor device.

Nowadays, organic electronic devices are attracting growing interest because of their characteristics such as flexibility. Examples of organic electronic devices include organic EL devices, organic solar cell elements, organic photoelectric transducers, organic transistor elements, etc. These organic electronic devices can be fabricated by a printing process (a solution process) in a short time at a low cost, hence attracting a great deal of expectation.

In the printing process for fabrication of organic electronic devices, an organic semiconductor material which contains an organic semiconductor compound needs to be soluble in a solvent. A semiconductor thin film is formed by application/printing of a solution containing the organic semiconductor material, followed by drying of the solvent. As the soluble low-molecular semiconductor material, condensed polycyclic aromatic compounds each containing a sulfur atom and/or a selenium atom have already been investigated. In an attempt to solubilize organic semiconductor materials, alkyl chains are introduced into long molecular axes of benzodithiophene (BDT) compounds, benzothieno-benzothiophene (BTBT) compounds, dinaphthodithiophene (DNTT) compounds, and the like (PTL 1 to PTL 3, and NPL 1 to NPL 4). In the case where an alkyl derivative of BTBT is employed, a resulting semiconductor material has sufficient solubility in an organic solvent to form a semiconductor thin film by a printing process. However, as a problem, since the number of fused rings is small relative to the number of alkyl chains, this semiconductor material tends to undergo a phase transition at low temperatures, and degrades heat resistance of organic electronic devices. In the case of an alkyl derivative of DNTT which has an increased number of fused rings, a resulting semiconductor material improves heat resistance of organic electronic devices, but its solubility in an organic solvent is insufficient to form a semiconductor thin film by a printing process. In view of these problems, there has been a demand for developing an organic semiconductor material (an organic semiconductor compound) in which an increase in the number of fused rings is compatible with sufficient solubility for a printing process, and which provides an organic electronic device having high heat resistance.

A recent report teaches an organic semiconductor material having a benzothieno-naphthothiophene (BTNT) skeleton in which the number of fused rings is greater than that of BTBT but fewer than that of DNTT (PTL 4 and PTL5).

Nevertheless, PTL 4 focuses on improvement in mobility and on/off ratio of an organic transistor, and storage stability, and merely exemplifies characteristics of an organic transistor obtained by a printing process in which a chloroform solution of a compound having a BTNT skeleton is applied to a substrate at 80°C. PTL 5 focuses on improvement in carrier mobility and electric current modulation characteristics of a thin-film semiconductor obtained by a treatment in a solution phase at a temperature below about 50°C, and merely exemplifies characteristics of an organic transistor obtained by spin coating at or around room temperature (<40°C) with a solution containing a compound having a BTNT skeleton. Neither PTL 4 nor PTL 5 provides any specific description about solubility of a compound having a BTNT skeleton at room temperature, and neither makes any reference to storage stability of a solution which contains a compound having a BTNT skeleton. Furthermore, PTL 4 and PTL 5 mention nothing about heat resistance sufficient to endure the steps for producing organic electronic devices.

### Citation List

#### Patent Literature

PTL 1: JP 2008-258592 A
PTL 2: WO 2008/047896 A1
PTL 3: WO 2010/098372 A1
PTL 4: JP 2010-34450 A
PTL 5: JP 2014-531435 A

#### Non-Patent Literature

NPL 1: Chemistry Letters, 2008, Vol. 37, No. 3, pp. 284-285
NPL 2: Journal of the American Chemical Society, 2007, Vol. 129, Issue 51, pp. 15732-15733

NPL 3: Advanced Materials, 2011, Vol. 23, Issue 10, pp. 1222-1225
NPL 4: Nature Communications, April 10, 2015, Vol. 6, Article number 6828

## Summary of Invention

### Technical Problem

An object of the present invention is to provide: an organic compound which has excellent solubility in an organic solvent at room temperature, excellent storage stability in a solution state, and excellent heat resistance; an organic semiconductor material and an organic semiconductor composition each containing the organic compound; an organic thin film which is obtainable by a printing process at room temperature using the organic semiconductor material and a method for producing the same; and an organic semiconductor device containing the organic thin film and having high mobility and high heat resistance.

### Solution to Problem

Specifically, the present invention relates to the following:

[1] an organic compound represented by General formula (A),

[Chemical Formula 1]

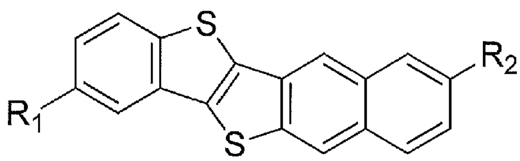

(A)

wherein one of $R_1$ and $R_2$ represents an alkyl group, an aromatic hydrocarbon group having an alkyl group, or a heterocyclic group having an alkyl group, and another of $R_1$ and $R_2$ represents an aliphatic hydrocarbon group, an aromatic hydrocarbon group, or a heterocyclic group, with a proviso that $R_1$ and $R_2$ are not alkyl groups simultaneously;
[2] the organic compound according to Item [1] above, wherein one of $R_1$ and $R_2$ is an aromatic hydrocarbon group which has an alkyl group having 2 to 16 carbon atoms, or a heterocyclic group which has an alkyl group having 2 to 16 carbon atoms;
[3] the organic compound according to Item [2] above, wherein one of $R_1$ and $R_2$ is an aromatic hydrocarbon group which has an alkyl group having 4 to 12 carbon atoms or a heterocyclic group which has an alkyl group having 4 to 12 carbon atoms, and another of $R_1$ and $R_2$ is an aromatic hydrocarbon group or a heterocyclic group;
[4] the organic compound according to Item [1] above, wherein one of $R_1$ and $R_2$ is a straight-chain alkyl group having 4 to 10 carbon atoms, and another of $R_1$ and $R_2$ is an aromatic hydrocarbon group or a heterocyclic group;
[5] the organic compound according to Item [1] above, wherein one of $R_1$ and $R_2$ is a branched-chain alkyl group having 6 to 12 carbon atoms, and another of $R_1$ and $R_2$ is an aromatic hydrocarbon group or a heterocyclic group;
[6] the organic compound according to any one of Items [1] to [5] above, wherein no peak indicating a phase transition is observed below 150°C in differential scanning calorimetry of the organic compound;
[7] the organic compound according to any one of Items [1] to [6] above, having solubility in an organic solvent at 25°C is 0.3 mass% or higher;
[8] the organic compound according to Item [7] above, wherein the organic solvent is a non-halogenated organic solvent;
[9] an organic semiconductor material which includes the organic compound according to any one of Items [1] to [8] above;
[10] an organic thin film which includes the organic semiconductor material according to Item [9] above;
[11] an organic semiconductor composition which contains the organic compound according to any one of Items [1] to [8] above and an organic solvent;
[12] the organic semiconductor composition according to Item [11] above, wherein a content of the organic compound is 0.1 to 5 mass%;
[13] a method for producing an organic thin film including the steps of applying or printing the organic semiconductor composition according to Item [11] or [12] above, and drying the composition;

[14] an organic semiconductor device which includes the organic thin film according to Item [10] above; and
[15] the organic semiconductor device according to Item [14] above, wherein the device is an organic transistor.

Advantageous Effects of Invention

An organic compound according to the present invention and an organic semiconductor material according to the present invention containing the same have good solubility in an organic solvent at room temperature, good storage stability in a solution state, and good heat resistance, and are therefore suitable for use in a printing process for producing an organic semiconductor device. An organic semiconductor composition according to the present invention containing the organic compound and an organic solvent has good solubility at room temperature, good storage stability, and good heat resistance, and is therefore suitable for use in a printing process for producing an organic semiconductor device. An organic thin film according to the present invention, obtained with use of the organic compound, can be employed to provide an organic semiconductor device according to the present invention having excellent mobility and excellent heat resistance. A method for producing the organic thin film according to the present invention enables production of the organic thin film for constituting the organic semiconductor device having excellent mobility and heat resistance.

Brief Description of Drawings

Figs. 1(a) to 1(f) are schematic views showing exemplary structures of organic transistors in embodiments of the present invention: Fig. 1(a) concerns a bottom-contact bottom-gate organic transistor; Fig. 1(b) concerns a top-contact bottom-gate organic transistor; Fig. 1(c) concerns a top-contact top-gate organic transistor; Fig. 1(d) concerns a top/bottom-contact bottom-gate organic transistor; Fig. 1(e) concerns a static induction organic transistor; and Fig. 1(f) concerns a bottom-contact top-gate organic transistor.
Figs. 2(a) to 2(f) are schematic views which illustrate the steps for producing an organic transistor in an embodiment of the present invention.
Fig. 3 represents transmission characteristics and output characteristics of an organic transistor in Example 6.

Description of Embodiments

The present invention is hereinafter described in detail.
An organic compound according to the present invention has a structure represented by Formula (A) below.

[Chemical Formula 2]

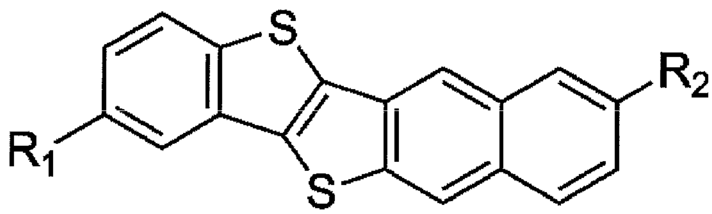

(A)

In Formula (A), one of $R_1$ and $R_2$ represents an alkyl group, an aromatic hydrocarbon group having an alkyl group, or a heterocyclic group having an alkyl group, and the other one of $R_1$ and $R_2$ represents an aliphatic hydrocarbon group, an aromatic hydrocarbon group, or a heterocyclic group, with a proviso that $R_1$ and $R_2$ are not alkyl groups simultaneously. The "aromatic hydrocarbon group or heterocyclic group represented by the other one of $R_1$ and $R_2$" may have a substituent.
The alkyl group represented by $R_1$ or $R_2$ in Formula (A) may be a straight- or branched-chain alkyl group, or an alicyclic alkyl group. Specific examples of the alkyl group include a methyl group, an ethyl group, a propyl group, an isopropyl group, an *n*-butyl group, an isobutyl group, an allyl group, a *t*-butyl group, an *n*-pentyl group, an *n*-hexyl group, an *n*-octyl group, an *n*-decyl group, an *n*-dodecyl group, an *n*-tridecyl group, an *n*-tetradecyl group, an *n*-cetyl group, an *n*-heptadecyl group, an *n*-butenyl group, a 2-ethylhexyl group, a 3-ethylheptyl group, a 4-ethyloctyl group, a 2-butyloctyl group, a 3-butylnonyl group, a 4-butyldecyl group, a 2-hexyldecyl group, a 3-octylundecyl group, a 4-octyldodecyl group, a 2-octyldodecyl group, a 2-decyltetradecyl group, a cyclohexyl group, a cyclopentyl group, an adamantyl group, a norbornyl group, etc. The alkyl group is preferably a straight- or branched-chain alkyl group such as an *n*-butyl group, an *n*-hexyl group, an *n*-octyl group, an *n*-decyl group, an *n*-dodecyl group, an ethylhexyl group, an ethyloctyl group, a

butyloctyl group, or a hexyldecyl group, and is more preferably an *n*-hexyl group, an *n*-octyl group, an *n*-decyl group, a 2-ethylhexyl group, a 3-ethylhexyl group, a 3-ethyloctyl group, or a 3-butyloctyl group.

The alkyl group represented by $R_1$ or $R_2$ in Formula (A) is preferably a straight- or branched-chain alkyl group having 2 to 16 carbon atoms, more preferably a straight- or branched-chain alkyl group having 4 to 12 carbon atoms, further preferably a straight-chain alkyl group having 4 to 10 carbon atoms or a branched-chain alkyl group having 6 to 12 carbon atoms, particularly preferably a straight- or branched-chain alkyl group having 6 to 10 carbon atoms, and most preferably a straight-chain alkyl group having 6 to 10 carbon atoms.

In the aromatic hydrocarbon group having an alkyl group represented by $R_1$ or $R_2$ in Formula (A), the aromatic hydrocarbon group is a residue of an aromatic hydrocarbon from which a hydrogen atom has been removed. Specific examples of the aromatic hydrocarbon group are a phenyl group, a naphthyl group, an anthryl group, a phenanthryl group, a pyrenyl group, a benzopyrenyl group, etc.

In the aromatic hydrocarbon group having an alkyl group represented by $R_1$ or $R_2$ in Formula (A), the aromatic hydrocarbon group is preferably a phenyl group or a naphthyl group, and is more preferably a phenyl group.

In the aromatic hydrocarbon group having an alkyl group represented by $R_1$ or $R_2$ in Formula (A), the alkyl group is similar to the alkyl group represented by $R_1$ or $R_2$ in Formula (A), and is preferably a straight- or branched-chain alkyl group having 1 to 10 carbon atoms, more preferably a straight- or branched-chain alkyl group having 1 to 6 carbon atoms, and further preferably a straight-chain alkyl group having 1 to 6 carbon atoms.

In the aromatic hydrocarbon group having an alkyl group represented by $R_1$ or $R_2$ in Formula (A), the substitution position of the alkyl group in the aromatic hydrocarbon group is not particularly limited. For example, in the case where the aromatic hydrocarbon group is a phenyl group, the preferable substitution position of the alkyl group is 4-position.

In the heterocyclic group having an alkyl group represented by $R_1$ or $R_2$ in Formula (A), the heterocyclic group is a residue of a hetero ring from which a hydrogen atom has been removed. Specific examples of the heterocyclic group are a pyridyl group, a pyrazyl group, a pyrimidyl group, a quinolyl group, an isoquinolyl group, a pyrrolyl group, an indolenyl group, an imidazolyl group, a carbazolyl group, a thienyl group, a furyl group, a pyranyl group, a pyridonyl group, a benzoquinolyl group, an anthraquinolyl group, a benzothienyl group, a benzofuryl group, a thienothienyl group, etc.

In the heterocyclic group having an alkyl group represented by $R_1$ or $R_2$ in Formula (A), the heterocyclic group is preferably a pyridyl group, a thienyl group, a benzothienyl group or a thienothienyl group, more preferably a thienyl group or a benzothienyl group, and further preferably a thienyl group.

In the heterocyclic group having an alkyl group represented by $R_1$ or $R_2$ in Formula (A), the alkyl group is similar to the alkyl group represented by $R_1$ or $R_2$ in Formula (A), and is preferably a straight- or branched-chain alkyl group having 1 to 10 carbon atoms, more preferably a straight- or branched-chain alkyl group having 4 to 8 carbon atoms, and further preferably a straight-chain alkyl group having 4 to 8 carbon atoms.

The aliphatic hydrocarbon group represented by $R_1$ or $R_2$ in Formula (A) (the aliphatic hydrocarbon group represented by the other one of $R_1$ and $R_2$) may be a saturated or unsaturated, straight-/branched-chain or cyclic hydrocarbon group, preferably having 1 to 30 carbon atoms, more preferably 1 to 20 carbon atoms, and further preferably 4 to 16 carbon atoms. Specific examples of the saturated or unsaturated, straight-/branched-chain or cyclic aliphatic hydrocarbon group include a methyl group, an ethyl group, a propyl group, an isopropyl group, an *n*-butyl group, an isobutyl group, an allyl group, a *t*-butyl group, an *n*-pentyl group, an *n*-hexyl group, an *n*-octyl group, an *n*-decyl group, an *n*-dodecyl group, an *n*-tridecyl group, an *n*-tetradecyl group, an *n*-cetyl group, an *n*-heptadecyl group, an *n*-butenyl group, a 2-ethylhexyl group, a 3-ethylheptyl group, a 4-ethyloctyl group, a 2-butyloctyl group, a 3-butylnonyl group, a 4-butyldecyl group, a 2-hexyldecyl group, a 3-octylundecyl group, a 4-octyldodecyl group, a 2-octyldodecyl group, a 2-decyltetradecyl group, a cyclohexyl group, a cyclopentyl group, an adamantyl group, a norbornyl group, etc. The saturated or unsaturated, straight-/branched-chain or cyclic, aliphatic hydrocarbon group is preferably a saturated, straight- or branched-chain alkyl group such as an *n*-butyl group, an *n*-hexyl group, an *n*-octyl group, an *n*-decyl group, an *n*-dodecyl group, an ethylhexyl group, an ethyloctyl group, a butyloctyl group, or a hexyldecyl group, and is more preferably an *n*-hexyl group, an *n*-octyl group, an *n*-decyl group, a 2-ethylhexyl group, a 3-ethylhexyl group or a 3-ethyloctyl group.

The aromatic hydrocarbon group represented by $R_1$ or $R_2$ in Formula (A) (the aromatic hydrocarbon group represented by the other one of $R_1$ and $R_2$) may be a phenyl group, a naphthyl group, an anthryl group, a phenanthryl group, a pyrenyl group, a benzopyrenyl group, etc., being preferably a phenyl group, a naphthyl group or a pyridyl group, more preferably a phenyl group or a naphthyl group, and further preferably a phenyl group.

The aromatic hydrocarbon group represented by $R_1$ or $R_2$ in Formula (A) (the aromatic hydrocarbon group represented by the other one of $R_1$ and $R_2$) may have a substituent. Examples of the substituent include the above-mentioned alkyl groups and the like, preferably the straight- or branched-chain alkyl groups having 1 to 6 carbon atoms, and more preferably the straight-chain alkyl groups having 1 to 6 carbon atoms.

The heterocyclic group represented by $R_1$ or $R_2$ in Formula (A) (the heterocyclic group represented by the other one of $R_1$ and $R_2$) may be a pyridyl group, a pyrazyl group, a pyrimidyl group, a quinolyl group, an isoquinolyl group, a pyrrolyl group, an indolenyl group, an imidazolyl group, a carbazolyl group, a thienyl group, a furyl group, a pyranyl

group, a pyridonyl group, a benzoquinolyl group, an anthraquinolyl group, a benzothienyl group, a benzofuryl group, etc., being preferably a pyridyl group, a thienyl group or a benzothienyl group, and more preferably a thienyl group or a benzothienyl group.

The heterocyclic group represented by $R_1$ or $R_2$ in Formula (A) (the heterocyclic group represented by the other one of $R_1$ and $R_2$) may have a substituent. Examples of the substituent include the above-mentioned alkyl groups and the like, preferably the straight- or branched-chain alkyl groups having 1 to 6 carbon atoms, and more preferably the straight-chain alkyl groups having 1 to 6 carbon atoms.

The organic compound represented by Formula (A) according to the present invention is preferably a combination of a preferable $R_1$ and a preferable $R_2$ mentioned above, and is more preferably a combination of a more preferable $R_1$ and a more preferable $R_2$.

Specifically, in a preferable organic compound represented by Formula (A), one of $R_1$ and $R_2$ is a phenyl group which has a straight- or branched-chain alkyl group having 1 to 10 carbon atoms or a straight- or branched-chain alkyl group having 1 to 16 carbon atoms, and the other one of $R_1$ and $R_2$ is a phenyl, pyridyl, thienyl or benzothienyl group which may have a straight- or branched-chain alkyl group having 1 to 6 carbon atoms. In a more preferable organic compound, one of $R_1$ and $R_2$ is a straight- or branched-chain alkyl group having 4 to 16 carbon atoms, and the other one of $R_1$ and $R_2$ is a phenyl, thienyl or benzothienyl group which may have a straight- or branched-chain alkyl group having 1 to 6 carbon atoms. In a further preferable organic compound, one of $R_1$ and $R_2$ is a straight- or branched-chain alkyl group having 4 to 12 carbon atoms, and the other one of $R_1$ and $R_2$ is a phenyl or benzothienyl group which may have a straight- or branched-chain alkyl group having 1 to 6 carbon atoms. In a particularly preferable organic compound, one of $R_1$ and $R_2$ is a straight-chain alkyl group having 4 to 10 carbon atoms or a branched-chain alkyl group having 6 to 12 carbon atoms, and the other one of $R_1$ and $R_2$ is a phenyl group which may have a straight- or branched-chain alkyl group having 1 to 6 carbon atoms. In the most preferable organic compound, one of $R_1$ or $R_2$ is a straight-chain alkyl group having 6 to 10 carbon atoms, and the other one of $R_1$ and $R_2$ is a phenyl group.

The organic compound represented by Formula (A) can be synthesized by application of a method, for example, as disclosed in JP 2011-256144 A.

Specific examples of the organic compound represented by Formula (A) according to the present invention are given below, but these examples should not be construed as limiting the present invention.

[Chemical Formula 3]

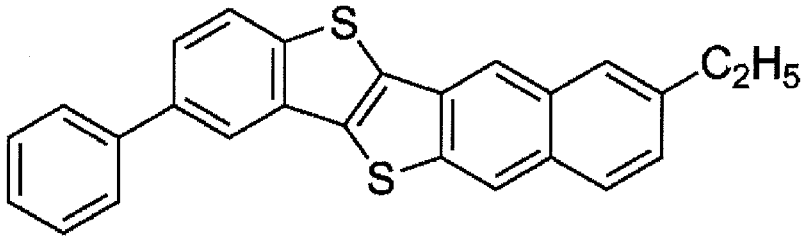

No.1

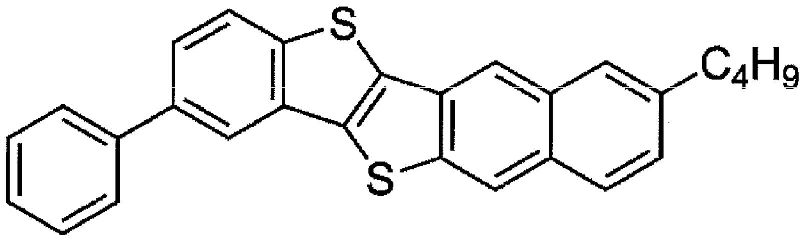

No.2

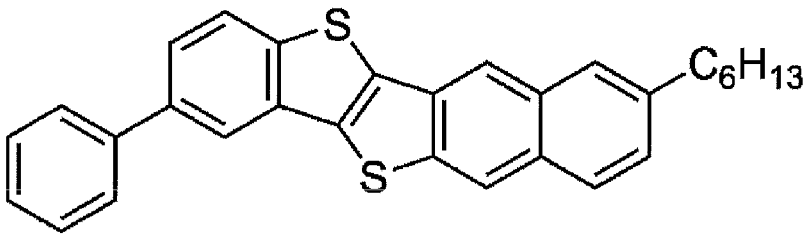

No.3

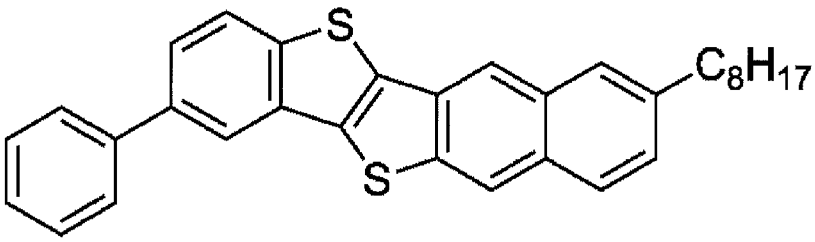

No.4

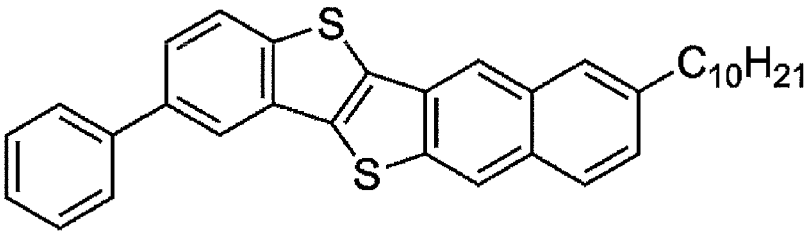

No.5

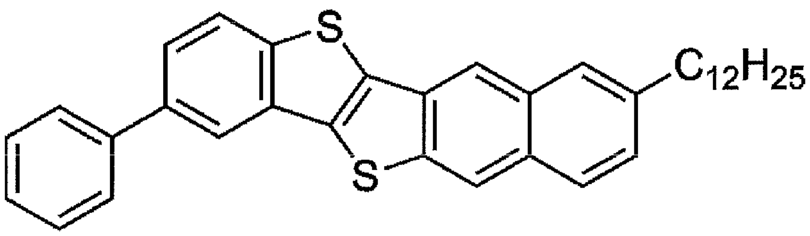

No.6

[Chemical Formula 4]

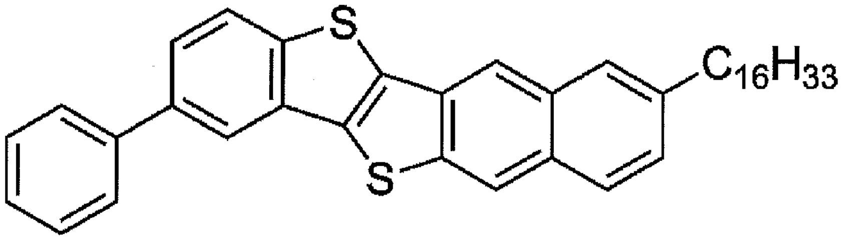

No.7

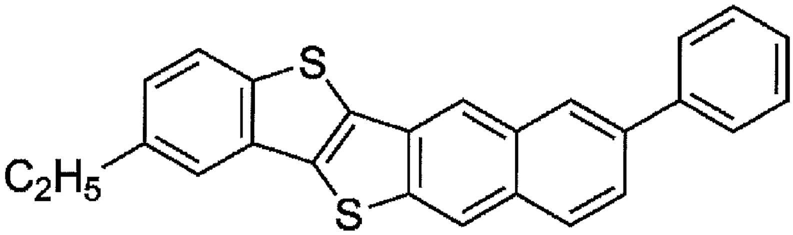

No.8

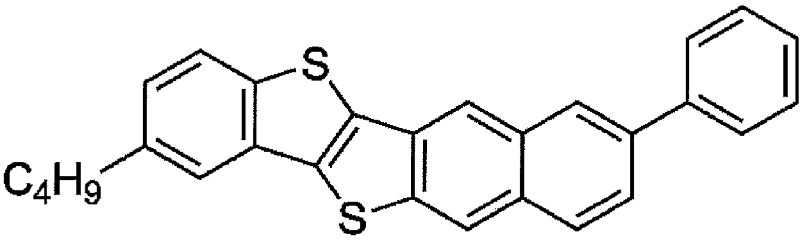

No.9

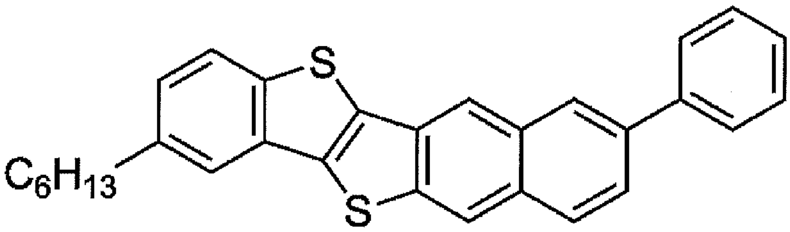

No.10

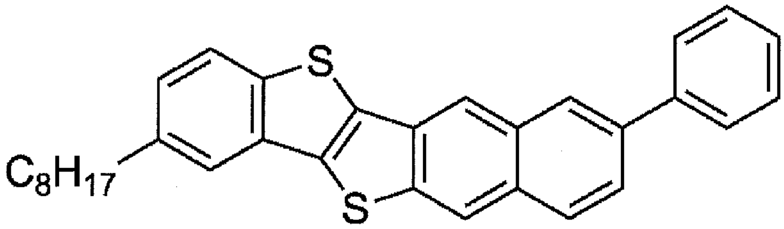

No.11

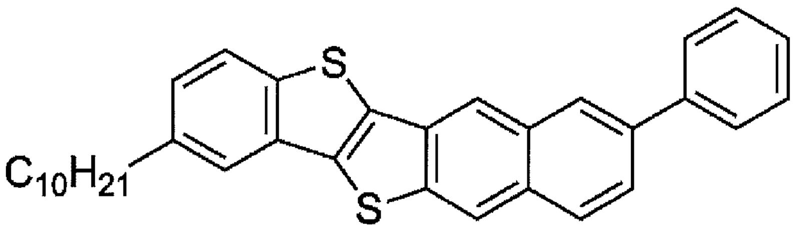

No.12

[Chemical Formula 5]

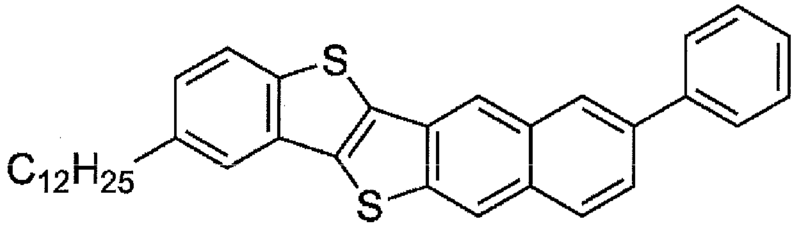

No.13

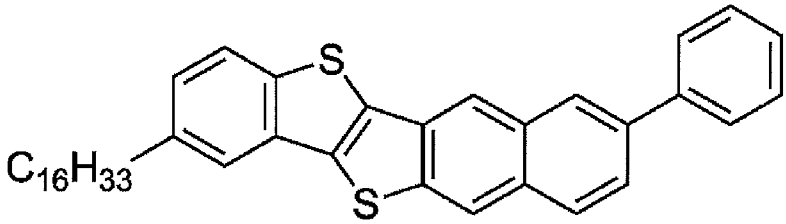

No.14

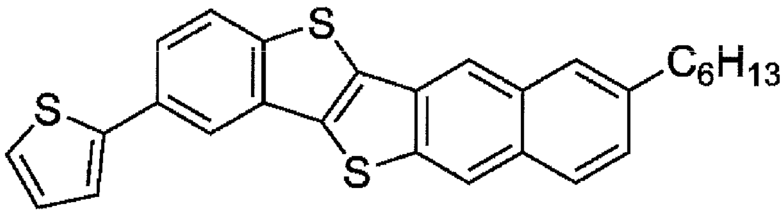

No.15

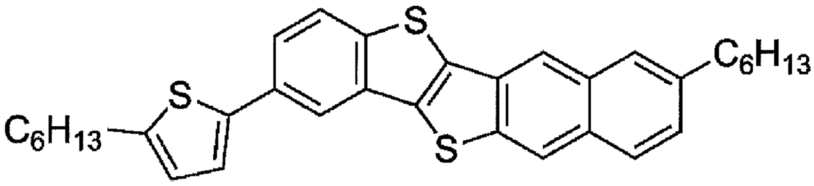

No.16

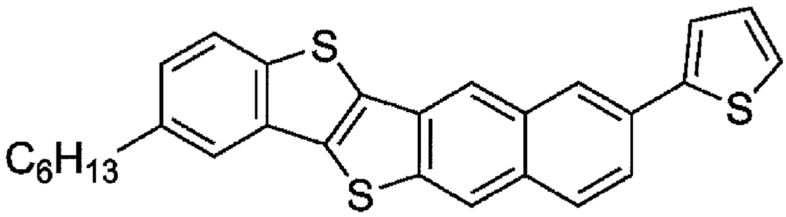

No.17

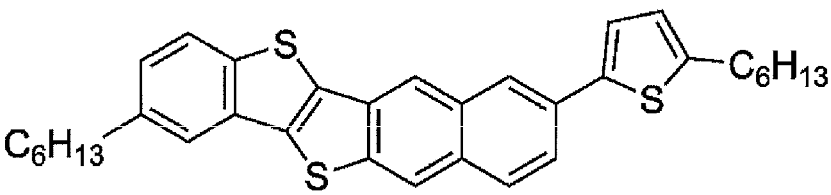

No.18

[Chemical Formula 6]

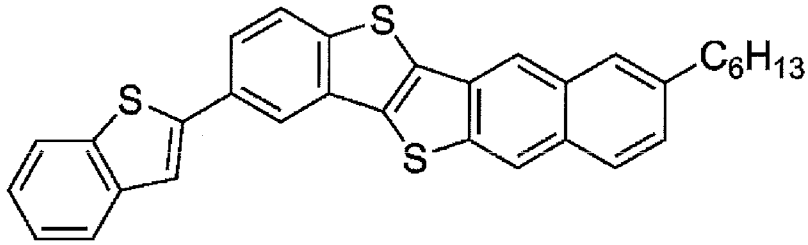

No.19

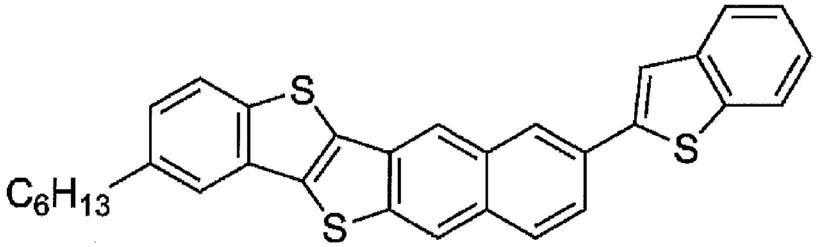

No.20

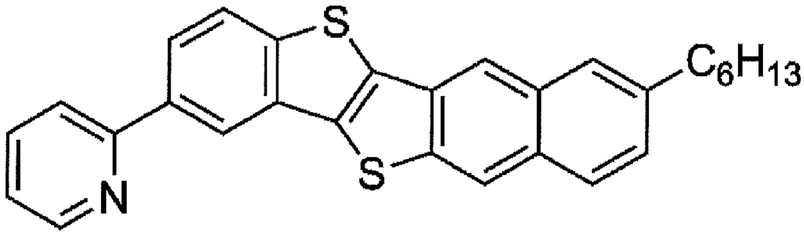

No.21

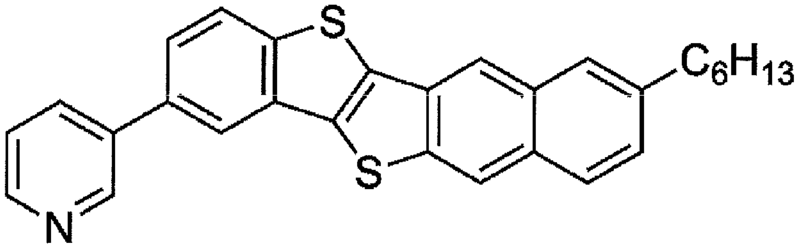

No.22

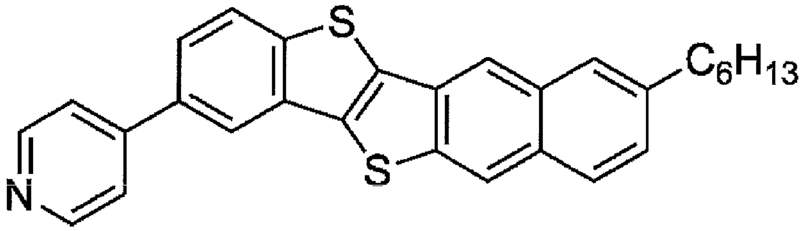

No.23

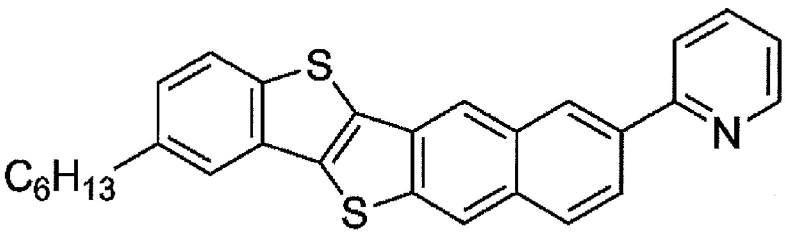

No.24

[Chemical Formula 7]

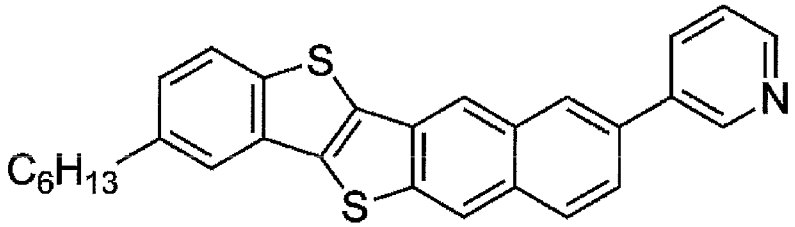

No.25

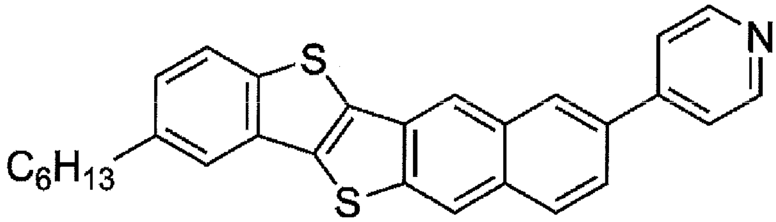

No.26

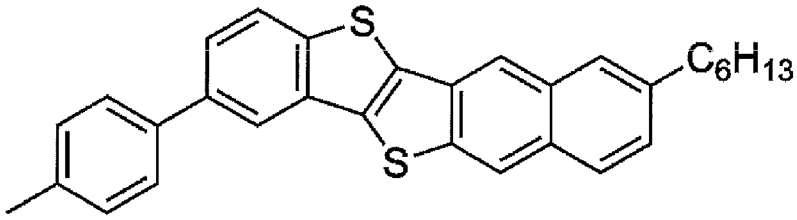

No.27

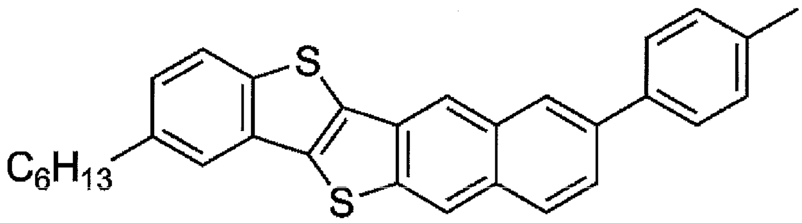

No.28

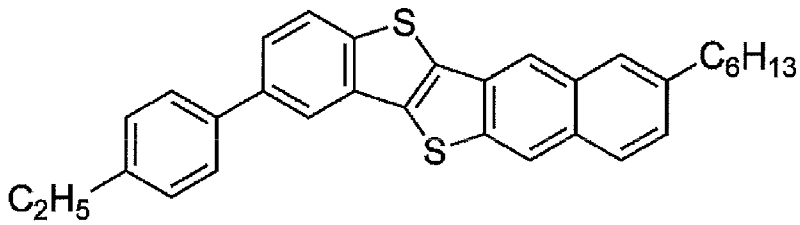

No.29

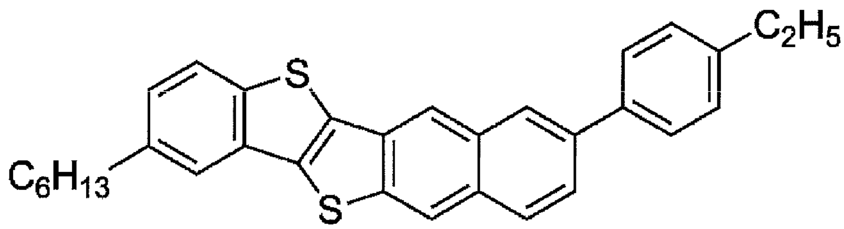

No.30

[Chemical Formula 8]

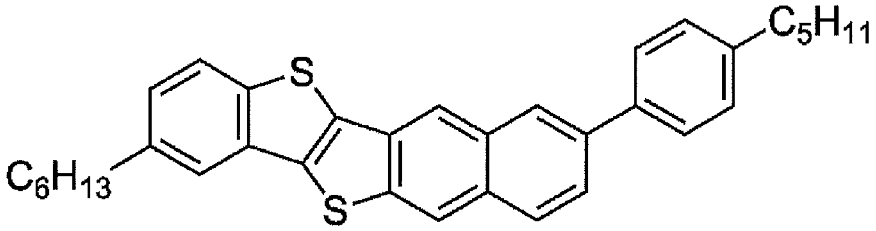

No.31

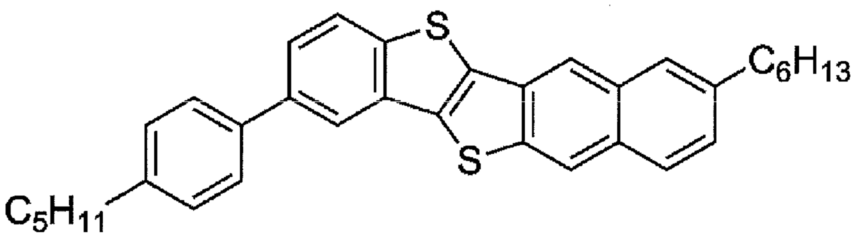

No.32

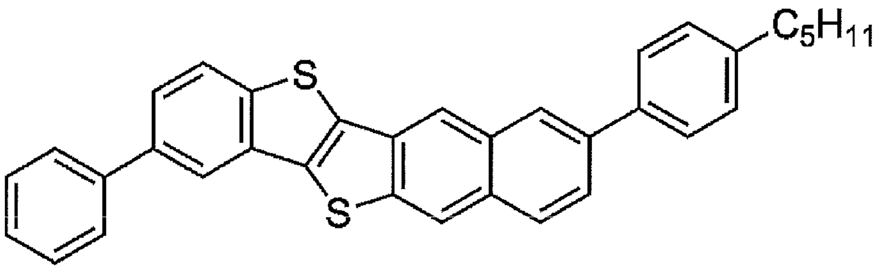

No.33

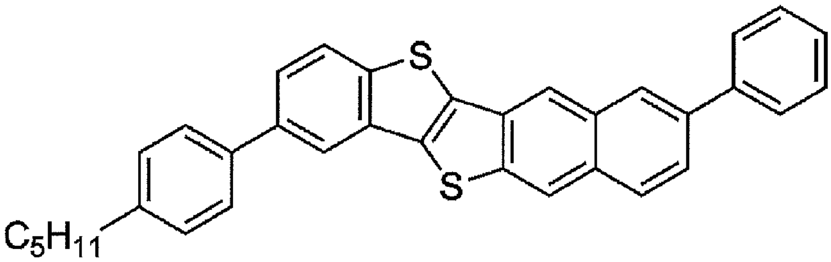

No.34

The organic compound represented by Formula (A) is characterized in not having a phase transition point below 150°C. A phase transition point indicates a temperature at which a substance changes from one phase to another, such as a liquid crystal transition point, a melting point, or a sublimation point. Generally, the phase transition temperature is represented by an endothermic peak in differential scanning calorimetry. In particular, a higher transition temperature is preferable for a melting point at which a substance changes from the solid phase to the liquid phase, and also for a transition temperature for a phase change which involves a great volume change concerning a nematic phase, a smectic A phase, and the like. For the compound of the present invention, the phase transition temperature is not below 150°C, preferably not below 170°C, more preferably not below 180°C, and further preferably not below 200°C.

The organic compound represented by Formula (A) is required to be soluble in an organic solvent. The organic solvent is not particularly limited as far as being capable of dissolving the organic compound represented by Formula (A). Considering environmental problems and other conditions, the organic solvent is preferably and practically a non-halogenated solvent. Further, considering the stability of a solution obtained by dissolving the organic compound represented by Formula (A) in the organic solvent, the organic compound represented by Formula (A) needs to have reasonably high solubility at room temperature. In the organic solvent, the solubility of the organic compound represented by Formula (A) at 25°C is usually 0.1 mass% or higher, preferably 0.3 mass% or higher, more preferably 0.5 mass% or higher, and further preferably 0.7 mass% or higher. In chloroform, the solubility of the organic compound represented by Formula (A) at 25°C is usually 0.1 mass% or higher, preferably 0.3 mass% or higher, more preferably 0.5 mass% or higher, and further preferably 0.7 mass% or higher. In toluene, the solubility of the organic compound represented by Formula (A) at 25°C is usually 0.1 mass% or higher, preferably 0.3 mass% or higher, more preferably 0.5 mass% or higher, and further preferably 0.7 mass% or higher. In tetrahydronaphthalene, the solubility of the organic compound represented by Formula (A) at 25°C is usually 0.1 mass% or higher, preferably 0.3 mass% or higher, more preferably 0.5 mass% or higher, and further preferably 0.7 mass% or higher. Regarding the stability of the solution, it is preferable

that crystals do not precipitate for 24 hours after dissolution, and it is more preferable that the compound remains completely dissolved for one week after dissolution.

As the organic solvent, there may be used halogenated solvents such as chloroform, dichloromethane, chlorobenzene, and dichlorobenzene, but use of non-halogenated solvents is preferable. Non-halogenated solvents include: aromatic hydrocarbons such as benzene, toluene, xylene, mesitylene, ethylbenzene, tetrahydronaphthalene, and cyclohexylbenzene; ethers such as diethyl ether, tetrahydrofuran, anisole, phenetole, and butoxybenzene; amides such as dimethylacetamide, dimethylformamide, and N-methylpyrrolidone; ketones such as acetone, methyl ethyl ketone, methyl isobutyl ketone, cyclopentanone, and cyclohexanone; nitriles such as acetonitrile, propionitrile, and benzonitrile; alcohols such as methanol, ethanol, isopropanol, butanol, and cyclohexanol; esters such as ethyl acetate, butyl acetate, ethyl benzoate, and diethyl carbonate; and hydrocarbons such as hexane, octane, decane, cyclohexane, and decalin.

The organic semiconductor material containing the organic compound represented by Formula (A) according to the present invention is suitable as a material for an organic thin film for organic electronic devices such as an organic EL device, an organic solar cell element, an organic photoelectric transducer, and an organic transistor device. In the organic semiconductor material, the content of the organic compound represented by Formula (A) is not particularly limited, but is usually not less than about 50 mass% and not greater than 100 mass%.

The organic semiconductor composition according to the present invention is obtained by dissolving or dispersing the organic semiconductor material containing the organic compound represented by Formula (A) into the organic solvent. The organic solvent may be a single organic solvent or a mixture of organic solvents.

In the organic semiconductor composition, the content of the organic compound represented by Formula (A) depends on the species of the organic solvent and the film thickness of the organic thin film to be formed. Relative to the organic solvent, the content of the organic compound is usually 0.1 to 5 mass%, preferably 0.3 to 5 mass%, and more preferably 0.5 mass% or more. The organic semiconductor composition according to the present invention is acceptable as far as being dissolved or dispersed in the organic solvent, but is preferable if forming a homogeneous solution in a dissolved state.

In order to improve the characteristics of the organic transistor, to provide other characteristics, or for other purposes, the organic semiconductor composition according to the present invention may contain not only the organic compound represented by Formula (A) and the organic solvent but also other additives, as required. The additive is not particularly limited unless inhibiting the function as a semiconductor. Examples of the additive include semiconductor materials having other structures, insulating materials, surfactants for rheology control, thickening agents, dopants for carrier injection or for regulation of carrier amount, and the like. These additives may be high-molecular or low-molecular unless inhibiting the stability as the composition. The content of such additives, which depends on the purposes and which thus cannot be generalized, is preferably less than the content of the organic compound represented by Formula (A).

An organic thin film can be formed by using the organic semiconductor material containing the organic compound represented by Formula (A) according to the present invention. The film thickness of the organic thin film, which depends on its intended use, is usually from 0.1 nm to 10 μm, preferably from 0.5 nm to 3 μm, and more preferably from 1 nm to 1 μm.

The organic thin film may be formed by dry processes (e.g. vapor deposition) or various solution processes, among which solution processes are preferred. The solution processes include, for example, spin coating, drop casting, dip coating, spraying, relief printing (such as flexography and resin relief printing), planographic printing (such as offset printing, dry offset printing, and pad printing), intaglio printing (such as gravure printing), stencil printing (such as screen printing, mimeograph, and lithographic printing), ink-jet printing, microcontact printing, and a combination of these processes. In the case of a solution process, it is preferable to apply or print the organic semiconductor composition, and then to evaporate the solvent to form an organic thin film.

An organic semiconductor device can be fabricated by using the organic compound represented by Formula (A) as a material. As an example of the organic semiconductor device, an organic transistor is described in detail.

An organic transistor has two electrodes (a source electrode and a drain electrode) in contact with an organic semiconductor, and controls an electric current flowing between the two electrodes by a voltage applied to another electrode (a gate electrode).

Generally, a common structure among the organic transistors is the MIS (Metal-Insulator-Semiconductor) structure in which a gate electrode is insulated by an insulating film. The MIS structure using a metal oxide film as the insulating film is called the MOS structure. Additionally, the MES structure in which a gate electrode is formed via a Schottky barrier is also available. Of these structures, the MIS structure is the most common among the organic transistors.

Hereinafter, an organic transistor is described in greater detail by way of some exemplary embodiments shown in Fig. 1. It should be noted, however, that the present invention is not limited to these exemplary structures.

In Fig. 1, organic transistors 10A-10F of the exemplary embodiments are composed of source electrodes 1, semiconductor layers 2, drain electrodes 3, insulator layers 4, gate electrodes 5, and substrates 6. The arrangement of the layers and electrodes can be properly selected in accordance with intended use of the organic transistors. The organic transistors 10A-10D and 10F are called lateral transistors, in which current is conducted parallel to the substrates 6. The organic transistor 10A has a bottom-contact bottom-gate structure. The organic transistor 10B has a top-contact

bottom-gate structure. The organic transistor 10C has a top-contact top-gate structure in which a source electrode 1, a drain electrode 3, and an insulator layer 4 are provided on a semiconductor layer 2, and a gate electrode 5 is provided on the insulator layer 4. The organic transistor 10D has a top/bottom-contact bottom-gate structure. The organic transistor 10F has a bottom-contact top-gate structure. The organic transistor 10E is a diagrammatic representation of a vertical transistor, namely, a static induction transistor (SIT). This SIT conducts electric current in a planarly spreading manner and allows a volume of carriers 8 to move at a time. Besides, vertical arrangement of the source electrode 1 and the drain electrode 3 can reduce a distance between the electrodes, and thereby ensures a high-speed response. Hence, the SIT is preferable for use for conducting a great amount of electric current, fast switching, or other like use. In the organic transistor 10E shown in Fig. 1(e), the substrate is omitted, but is usually provided on an external side of the source electrode 1 or the drain electrode 3 of the organic transistor 10E.

Components in the exemplary embodiments are now described.

The substrate 6 is required to hold the layers formed thereon without delamination. For example, the substrate 6 may be made of an insulating material such as a resin plate, a resin film, paper, glass, quartz, or ceramics, may be prepared by forming an insulating layer on a conductive substrate such as a metal or alloy by coating or a like technique, or may be made of various combinations of materials (e.g. a resin with an inorganic material). Applicable resin films are made of, for example, polyethylene terephthalate, polyethylene naphthalate, polyethersulfone, polyamide, polyimide, polycarbonate, cellulose triacetate, polyether imide, etc. A substrate made of a resin film or paper imparts flexibility to the organic transistor, thereby providing a flexible, light-weight, and thus more useful organic transistor. The thickness of the substrate 6 is usually from 1 $\mu$m to 10 mm, and preferably from 5 $\mu$m to 5 mm.

The source electrode 1, the drain electrode 3, and the gate electrode 5 are made of conductive materials. For example, these electrodes may be made of: metals such as platinum, gold, silver, aluminum, chromium, tungsten, tantalum, nickel, cobalt, copper, iron, lead, tin, titanium, indium, palladium, molybdenum, magnesium, calcium, barium, lithium, potassium, and sodium, and alloys containing these metals; conductive oxides such as $InO_2$, $ZnO_2$, $SnO_2$, ITO, etc.; conductive polymer compounds such as polyaniline, polypyrrole, polythiophene, polyacetylene, poly(*p*-phenylene vinylene), and polydiacetylene; semiconductors such as silicon, germanium, and gallium arsenide; carbon materials such as carbon black, fullerene, carbon nanotube, graphite, and graphene. The conductive polymer compounds and the semiconductors may be doped. Examples of dopants include: inorganic acids such as hydrochloric acid and sulfuric acid; organic acids having an acidic functional group such as sulfonic acid; Lewis acids such as $PF_5$, $AsF_5$, and $FeCl_3$; halogen atoms such as iodine; metal atoms such as lithium, sodium, and potassium. Boron, phosphorus, and arsenic are frequently used as dopants for silicon and other inorganic semiconductors.

The electrodes may be also made of conductive composite materials in which carbon black and/or metal particles is/are dispersed on the above dopants. For the source electrode 1 and the drain electrode 3 which are in direct contact with the semiconductor, it is important to reduce a contact resistance by selecting a proper work function or performing surface treatment or the like.

A distance between the source electrode 1 and the drain electrode 3 (a channel length) is an important factor in determining characteristics of the organic transistor. Hence, a proper channel length is required. A short channel length can increase the amount of extractable electric current, but may trigger a short channel effect including an influence of a contact resistance and may deteriorate semiconductor properties. The channel length is usually from 0.01 to 300 $\mu$m, and preferably from 0.1 to 100 $\mu$m. A width between the source electrode 1 and the drain electrode 3 (a channel width) is usually from 10 to 5000 $\mu$m, and preferably from 40 to 2000 $\mu$m. The channel width can be further extended by making the source electrode 1 and the drain electrode 3 in a comb structure. A proper channel width needs to be determined in accordance with a required amount of electric current, the structure of the organic transistors 10A-10F, and other factors.

The next description is directed to the structure (the form) of the source electrode 1 and the drain electrode 3. The source electrode 1 and the drain electrode 3 may have an identical structure or different structures.

For the bottom-contact structure, it is generally preferable to fabricate the source electrode 1 and the drain electrode 3 in cuboid shapes by lithography. In various printing methods, printing precision has been improved so much as to enable precise fabrication of the source electrode 1 and the drain electrode 3 by ink-jet printing, gravure printing, screen printing, or the like. For the top-contact structure in which the source electrode 1 and the drain electrode 3 are provided on the semiconductor layer 2, the source electrode 1 and the drain electrode 3 can be fabricated by vapor deposition using a shadow mask or the like. It has also become possible to make the source electrode 1 and the drain electrode 3 by directly printing electrode patterns by ink-jet or other like techniques. The length of the source electrode 1 and the drain electrode 3 is the same as the channel width mentioned above. Although the width of the source electrode 1 and the drain electrode 3 is not particularly limited, a smaller width is preferable in order to minimize the device area while stabilizing electric characteristics. The width of the source electrode 1 and the drain electrode 3 is usually from 0.1 to 1000 $\mu$m, and preferably from 0.5 to 100 $\mu$m. The thickness of the source electrode 1 and the drain electrode 3 is usually from 0.1 to 1000 nm, preferably from 1 to 500 nm, and more preferably from 5 to 200 nm. The electrodes 1, 3, 5 are connected by wires, whose materials are substantially similar to those for the electrodes 1, 3, 5.

The insulator layer 4 is made of an insulating material. For example, the insulating material may be selected from: polymers such as polyparaxylylene, polyacrylates, poly(methyl methacrylate), polystyrene, polyvinylphenol, polyamides, polyimides, polycarbonates, polyesters, polyvinyl alcohol, polyvinyl acetate, polyurethane, polysulfone, polysiloxane, fluororesin, epoxy resins, and phenolic resins, and copolymers combining these polymers; metal oxides such as silicon oxide, aluminum oxide, titanium oxide, and tantalum oxide; ferroelectric metal oxides such as $SrTiO_3$ and $BaTiO_3$; dielectrics such as nitrides like silicon nitride and aluminum nitride, sulfides, and fluorides; or polymers in which particles of such dielectrics are dispersed. For the purpose of reducing a leakage current, the insulator layer 4 having high electrical insulation characteristics is preferable. Use of this preferable insulator layer 4 can make the film thinner, can increase an isolation capacitance, and can extract a greater amount of electric current. Additionally, in order to improve mobility of the semiconductor, it is preferable to decrease the surface energy on the surface of the insulator layer 4, and to provide a smooth film without unevenness. For this purpose, a self-assembled monolayer or two insulator layers may be formed. The film thickness of the insulator layer 4 depends on the material, but is usually from 0.1 nm to 100 $\mu$m, preferably from 0.5 nm to 50 $\mu$m, and more preferably from 1 nm to 10 $\mu$m.

The organic semiconductor material for the semiconductor layer 2 serving as the organic semiconductor layer may be at least one of the organic compounds represented by General formula (A). By the organic thin film forming process as described above, an organic thin film which contains an organic compound represented by General formula (A) is formed as the semiconductor layer 2.

The semiconductor layer 2 may be composed of a plurality of layers, but a single layer structure is more preferable. Preferably, the film thickness of the semiconductor layer 2 should be as thin as possible without sacrificing the required function of the semiconductor layer 2. In the case of the lateral organic transistor such as the organic transistors 10A, 10B, and 10D, the characteristics of the organic transistor do not depend on the film thickness as long as the film thickness of the semiconductor layer 2 is equal to or greater than a certain degree, but an increase in film thickness often causes an increase in leakage current. That is why a practically minimum thickness is preferred. The film thickness of the semiconductor layer 2 for presenting a required function is usually from 1 nm to 1 $\mu$m, preferably from 5 nm to 500 nm, and more preferably from 10 nm to 300 nm.

The organic transistor may include an additional layer, as required, for example, between the substrate 6 and the insulator layer 4, between the insulator layer 4 and the semiconductor layer 2, or on the external surface of the organic transistor. For example, a protective layer formed directly, or via another layer, on the semiconductor layer 2 can alleviate an influence of the external environment such as humidity. As another advantage, the protective layer can raise the on/off ratio of the organic transistor, and thus can stabilize the electrical characteristics.

The material for the protective layer is not particularly limited, but may preferably be, for example, resin films made of: epoxy resins, acrylic resins such as poly(methyl methacrylate), polyurethane, polyimide, polyvinyl alcohol, fluororesin, and polyolefin; inorganic oxide films such as silicon oxide, aluminum oxide, and silicon nitride; dielectric films such as nitride films; etc. A particularly preferable material is a resin (a polymer) whose oxygen transmission rate, moisture transmission rate, and water absorption coefficient are small. A protective gas barrier material developed for the organic EL display is also available. The film thickness of the protective layer can be freely selected to suit its purpose, and is usually from 100 nm to 1 mm.

Characteristics of the organic transistor can be also improved by advance surface modification or advance surface treatment to the substrate 6 or the insulator layer 4 on which the semiconductor layer 2 is to be deposited. For example, by controlling the level of hydrophilicity/hydrophobicity at the surface of the substrate 6, it is possible to improve the film quality and film forming property of the film formed thereon. In particular, characteristics of the organic semiconductor material may change significantly, depending on the film conditions such as molecular orientation. Hence, it is assumed that surface treatment to the substrate 6, the insulator layer 4, or a like component controls molecular orientation at an interface with the semiconductor layer 2 to be formed later, or reduces trap sites in the substrate 6 and the insulator layer 4, so that carrier mobility or other characteristics can be improved.

The trap site is a functional group (e.g. a hydroxyl group) in an untreated substrate 6. This functional group attracts an electron, and thereby causes a decrease in carrier mobility. Hence, in many cases, reduction of trap sites is also effective for improvement in carrier mobility and other characteristics.

The surface treatment for improving characteristics as mentioned above includes, for example: self-assembled monolayer treatment by hexamethyldisilazane, octyltrichlorosilane, octadecyltrichlorosilane, or the like; surface treatment by a polymer or the like; acid treatment by hydrochloric acid, sulfuric acid, acetic acid, or the like; alkali treatment by sodium hydroxide, potassium hydroxide, calcium hydroxide, ammonia, or the like; ozone treatment; fluorinating treatment; plasma treatment by oxygen, argon or the like; Langmuir-Blodgett film formation treatment; other film formation treatment for insulator thin films or semiconductor thin films; mechanical treatment; electrical treatment such as corona discharge; rubbing treatment using fibers or the like; and a combination of these treatments.

In these embodiments, the process for forming the constitutive layers such as the substrate 6, the insulator layer 4, and the semiconductor layer 2 can be suitably selected from the vacuum processes and the solution processes as mentioned above.

Next, referring to Fig. 2, a method for producing an organic transistor device according to the present invention is described by taking the top-contact bottom-gate organic transistor 10B shown in Fig. 1(b) as an example. This production method is similarly applicable to the organic transistors of the other embodiments mentioned above, and the like.

(The substrate 6 for the organic transistor and substrate treatment)

An organic transistor according to the present invention is fabricated by providing various layers and electrodes on the substrate 6, as required (see Fig. 2(a)). The substrate 6 as described above can be employed herein. The substrate 6 may be subjected to surface treatment or the like as mentioned above. The thickness of the substrate 6 is preferably as thin as possible without sacrificing its required function. The thickness of the substrate 6 depends on the material, but is usually from 1 μm to 10 mm, and preferably from 5 μm to 5 mm. Where necessary, the substrate may have an electrode function.

(Formation of the gate electrode 5)

The gate electrode 5 is formed on the substrate 6 (see Fig. 2(b)). The material for the gate electrode 5 is as described above. The gate electrode 5 can be formed by various film-forming methods including, for example, vacuum vapor deposition, sputtering, coating, thermal transfer, printing, sol-gel process, etc. Preferably, the gate electrode 5 is subjected to patterning into a desired shape, as required, during or after the film formation. The patterning can be conducted by various ways, including, for example, photolithography which combines patterning and etching of a photoresist. Other patterning techniques include vapor deposition using a shadow mask, sputtering, printing processes such as ink-jet printing, screen printing, offset printing, and relief printing, soft lithography techniques such as microcontact printing, and a combination of these techniques. The film thickness of the gate electrode 5 depends on the material, but is usually from 0.1 nm to 10 μm, preferably from 0.5 nm to 5 μm, and more preferably from 1 nm to 3 μm. In the case where the gate electrode 5 serves also as the substrate 6, the film thickness may be greater than the above values.

(Formation of the insulator layer 4)

The insulator layer 4 is formed on the gate electrode 5 (see Fig. 2(c)). The material for the insulator layer 4 is as described above. The insulator layer 4 can be formed by various methods including, for example: coating processes such as spin coating, spray coating, dip coating, casting, bar coating, and blade coating; printing processes such as screen printing, offset printing, and ink-jet printing; and dry processes such as vacuum vapor deposition, molecular beam epitaxy, ionized cluster beam deposition, ion plating, sputtering, atmospheric pressure plasma deposition, and CVD. Other available processes include a sol-gel process, a process of forming an oxide film on a metal by thermal oxidation (e.g. alumite on aluminum, or silicon oxide on silicone), and the like. Additionally, the insulator layer 4 may be subjected to a predetermined surface treatment at a portion to be in contact with the semiconductor layer 2, for desirable orientation of semiconductor-constituting molecules (e.g. molecules of the compound represented by General formula (A)) at the interface between the layers 2 and 4. The surface treatment techniques for the substrate 6 can be similarly applied to the insulator layer 4. The film thickness of the insulator layer 4 is preferably as thin as possible because the amount of extractable electricity can be increased by raising the capacitance. Given the fact that a thinner film increases a leakage current, the insulator layer 4 is preferably as thin as possible without sacrificing its function. The film thickness of the insulator layer 4 is usually from 0.1 nm to 100 μm, preferably from 0.5 nm to 50 μm, and more preferably from 5 nm to 10 μm.

(Formation of the semiconductor layer 2)

The organic semiconductor material containing the organic compound represented by General formula (A) according to the present invention is employed in formation of the semiconductor layer 2 (see Fig. 2(d)). The semiconductor layer 2 can be formed by various methods including, specifically: coating such as dip coating, die coating, roll coating, bar coating, and spin coating; and solution processes such as ink-jet printing, screen printing, offset printing, and microcontact printing.

Now, description is made of the method for obtaining the semiconductor layer 2 by forming a film by a solution process. To start with, the organic compound represented by General formula (A) according to the present invention is dissolved in a solvent or the like, and, where necessary, an additive or the like may be added thereto. The resulting composition is applied to the substrate (an exposed part of the insulator layer 4, the source electrode 1, and the drain electrode 3). The application method includes spin coating, drop casting, dip coating, spraying, relief printing (such as flexography and resin relief printing), planographic printing (such as offset printing, dry offset printing, and pad printing), intaglio printing (such as gravure printing), stencil printing (such as silk screen printing, mimeograph, and lithographic printing), ink-jet printing, microcontact printing, and a combination of these processes.

Other applicable methods analogous to the above application methods include: the Langmuir-Blodgett technique in which a monolayer film for the organic semiconductor layer is prepared by dropping the above composition on the water surface, transferred to the substrate, and deposited thereon; or a process in which a material in a liquid crystal state or melt state is sandwiched and introduced between two substrates by the capillary action.

The environment for the film formation such as the substrate temperature and the composition temperature is also important. Preferably, the substrate temperature and the composition temperature, which may affect the characteristics of the organic transistor, are selected carefully. The substrate temperature is usually from 0 to 200°C, preferably from 10 to 120°C, and more preferably from 15 to 100°C. Such temperatures heavily depend on the solvent in the composition or other factors, and thus require careful consideration.

The film thickness of the semiconductor layer 2 produced by this method is preferably as thin as possible without sacrificing its function. An increase in the film thickness of the semiconductor layer 2 is feared to increase a leakage current. The film thickness of the semiconductor layer 2 is usually from 1 nm to 1 μm, preferably from 5 nm to 500 nm, and more preferably from 10 nm to 300 nm.

The characteristics of the thus formed semiconductor layer 2 (see Fig. 2(d)) can be further improved by post-treatment. For example, heat treatment can improve and stabilize the characteristics of the organic semiconductor by alleviating distortion in the film that has been generated during the film formation, reducing pinholes and the like, controlling arrangement/orientation in the film, and more. In the fabrication of the organic transistor according to the present invention, this heat treatment is effective for improvement in the transistor characteristics. The heat treatment is conducted by heating the substrate after the formation of the semiconductor layer 2. The temperature of the heat treatment is not particularly limited, but is usually from the room temperature to about 150°C, preferably from 40 to 120°C, and more preferably from 45 to 100°C. The time for the heat treatment is not particularly limited, but is usually from about 10 seconds to about 24 hours, and preferably from about 30 seconds to about 3 hours. The heat treatment may be conducted in atmospheric atmosphere, or may be conducted in an inert atmosphere such as nitrogen or argon. As an additional post-treatment, the film shape or the like can be controlled by solvent vapor.

As other post-treatment processes for the semiconductor layer 2, treatment with use of oxidizing or reducing gas (such as oxygen or hydrogen) or oxidizing or reducing liquid can induce a change in the characteristics due to oxidation or reduction. This post-treatment may be performed, for example, in order to increase or decrease the carrier density in the film.

The characteristics of the semiconductor layer 2 can be also changed by a technique called doping, by adding a tiny amount of element, atomic group, molecule, or macromolecule to the semiconductor layer 2. The semiconductor layer 2 can be doped with, for example: oxygen, hydrogen, or acids such as hydrochloric acid, sulfuric acid, and sulfonic acid; Lewis acids such as $PF_5$, $AsF_5$ and $FeCl_3$; halogen atoms such as iodine; metal atoms such as sodium and potassium; donor compounds such as tetrathiafulvalene (TTF) and phthalocyanine. Doping of the semiconductor layer 2 can be conducted by bringing the above-mentioned gas into contact with the semiconductor layer 2, dipping the semiconductor layer 2 in a solution, or subjecting the semiconductor layer 2 to electrochemical doping. The timing of such doping is not limited to after the fabrication of the semiconductor layer 2, and may be during the synthesis of the organic compound. In the process where the semiconductor layer 2 is fabricated with use of the organic semiconductor composition, a doping material may be added to the organic semiconductor composition, or may be added in a step of forming the semiconductor layer 2. Doping may be further performed during the vapor deposition by coevaporation in which a doping material is added to the material for the semiconductor layer 2, may be performed by exposing the semiconductor layer 2 to the surrounding atmosphere for fabrication of the semiconductor layer 2 (namely, the semiconductor layer 2 is fabricated in the presence of a doping material), or may be even performed by accelerating ions in the vacuum and allowing the ions to collide with the film.

Effects of the doping include a change in electrical conductivity due to an increase or decrease in carrier density, a change in carrier polarity (p-type, n-type), a change in Fermi level, and the like.

(Formation of the source electrode and the drain electrode)

The process and other requirements for forming the source electrode 1 and the drain electrode 3 are similar to those for the gate electrode 5 (see Fig. 2(e)). In order to reduce the contact resistance with the semiconductor layer 2, various additives and the like can be employed.

(The protective layer 7)

The protective layer 7 formed on the semiconductor layer 2 is advantageous in minimizing the influence of the external air and stabilizing the electrical characteristics of the organic transistor (see Fig. 2(f)). The material for the protective layer 7 is as described above. The film thickness of the protective layer 7 can be freely selected to suit its purpose, and is usually from 100 nm to 1 mm.

The protective layer 7 can be formed by various methods. In the case where the protective layer 7 is made of a resin, the protective layer may be formed, for example, by a method in which a resin solution is applied and dried to give a resin film, or a method in which a resin monomer is applied or deposited, and then polymerized. Crosslinking treatment may be performed after the film formation. In the case where the protective layer 7 is made of an inorganic substance, the protective layer 7 may be also formed, for example, by a vacuum process such as sputtering or vapor deposition, or by a solution process such as a sol-gel process.

In addition to the protective layer 7 formed on the semiconductor layer 2, the organic transistor may have one or more additional protective layers 7, as required, between the layers. In some cases, the additional protective layer(s) can be effective for stabilization of the electrical characteristics of the organic transistor.

Since the organic compound represented by General formula (A) is employed as the organic semiconductor material, the organic transistor can be produced in a relatively low-temperature process. Hence, a flexible material such as a plastic plate or a plastic film, which is not applicable under a high temperature condition, can serve as the substrate 6. Eventually, it is possible to produce a light-weight break-proof organic transistor with excellent flexibility, and to use the organic transistor, for example, as a switching device for an active matrix for a display.

The organic transistor can be also employed as a digital device or an analog device such as a memory circuit device, a signal driver circuit device, or a signal processing circuit device. By combining these devices, it is further possible to produce a display, an IC card, an IC tag, etc. Further, the organic transistor can be employed as a sensor because its characteristics can be changed by an external stimulus, for example, by a chemical substance.

## Examples

Hereinafter, the present invention is described in detail by way of examples. It should be noted, however, the present invention is not limited to these examples.

In the following examples, reactions and measurements in an inert gas atmosphere were conducted with use of a solvent obtained by anhydrous distillation, and other reactions and operations were conducted with use of commercial Grade 1 or Special Grade solvents. The reagents were purified, as required, by anhydrous distillation or the like, and otherwise commercially available Grade 1 or Special Grade reagents were employed. In the examples, following analytical instrument and measurement instrument were employed. The nuclear magnetic resonance spectroscopy was conducted by means of LAMBDA-NMR (395.75 MHz, σ value, ppm, internal standard TMS). The differential scanning calorimetry was conducted by means of a differential scanning calorimeter METTLER-TOLED DSC1.

The organic compound represented by Formula (A) was synthesized in the examples as mentioned below.

Specifically, in Examples 1 to 3, the organic compound represented by Formula (A) was synthesized by a synthesis route shown below.

[Chemical Formula 9]

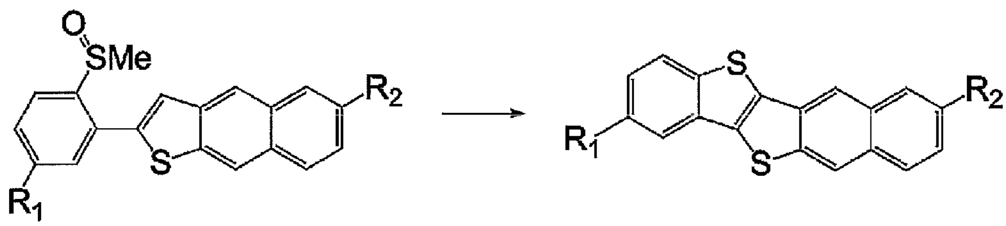

Example 1 (Synthesis of an organic compound according to the present invention, represented by No. 3 in the specific examples given above)

In a nitrogen atmosphere, 5.2 g (10.7 mmol) of 2-(2-methylsulfinyl-5-phenyl-1-phenyl)-6-hexylnaphtho[2,3-b]thiophene, 1.5 g of phosphorous pentoxide, and 150 mL of trifluoromethanesulfonic acid were added into a 300-mL eggplant-shaped flask, and stirred for six hours at room temperature. The reaction liquid was poured into ice water, and solid precipitate was filtered out. The filtered solid and 150 mL of pyridine were fed in a 300-mL eggplant-shaped flask, and allowed to react for three hours under reflux. After the reaction, the reaction mixture was cooled to room temperature, and the solid precipitate was filtered out to give 3.1 g of a yellow solid as Organic compound 1 according to the present invention. The yield of Organic compound 1 was 65%.

[Chemical Formula 10]

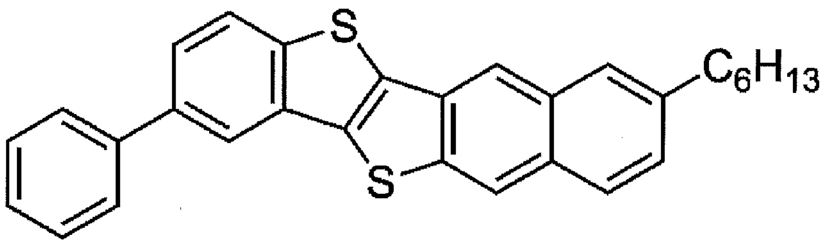

No.3

The measurement result of nuclear magnetic resonance spectroscopy for Organic compound 1 is given below. $^1$H-NMR (400 MHz, $CDCl_3$) δ0.89 (t, 3H), 1.24-1.42 (m, 6H), 1.71 (Quin, 2H), 2.81 (t, 2H), 7.36-7.41 (m, 2H), 7.50 (t, 2H), 7.66 (dd, 1H), 7.71-7.73 (m, 2H), 7.78 (s, 1H), 7.85 (d, 1H), 7.99 (d, 1H), 8.06 (d, 1H), 8.30 (s, 1H), 8.35 (s, 1H).

Example 2 (Synthesis of an organic compound according to the present invention, represented by No. 10 in the specific examples given above)

Except for using 2-(2-methylsulfinyl-5-hexyl-1-phenyl)-6-phenylnaphtho[2,3-b]thiophene instead of 2-(2-methylsulfinyl-5-phenyl-1-phenyl)-6-hexylnaphtho[2,3-b]thiophene, the procedure of Example 1 was followed to give 2.1 g of Organic compound 2 according to the present invention. The yield of Organic compound 2 was 43%.

[Chemical Formula 11]

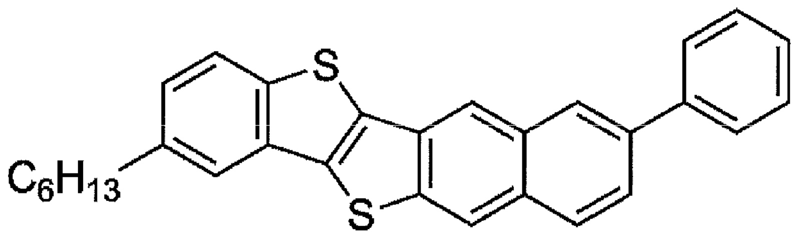

No.10

The measurement result of nuclear magnetic resonance spectroscopy for Organic compound 2 is given below. $^1$H-NMR (400 MHz, $CDCl_3$) δ0.91 (t, 3H), 1.32-1.42 (m, 6H), 1.73 (Quin, 4H), 2.80 (t, 4H), 7.28 (dd, 1H), 7.39-7.44 (m, 1H), 7.50-7.55 (m, 2H), 7.70 (d, 1H), 7.62-7.81 (m, 3H), 7.85 (d, 1H), 8.00 (d, 1H), 8.22 (d, 1H), 8.39 (s, 1H), 8.41 (s, 1H).

Example 3 (Synthesis of an organic compound according to the present invention, represented by No. 19 in the specific examples given above)

Except for using 2-[2-methylsulfinyl-5-(2-benzo[2,3-b]thienyl)-1-phenyl]-6-hexylnaphtho[2,3-b]thiophene instead of 2-(2-methylsulfinyl-5-phenyl-1-phenyl)-6-hexylnaphtho[2,3-b]thiophene, the procedure of Example 1 was followed to give 3.1 g of Organic compound 3 according to the present invention. The yield of Organic compound 3 was 58%.

[Chemical Formula 12]

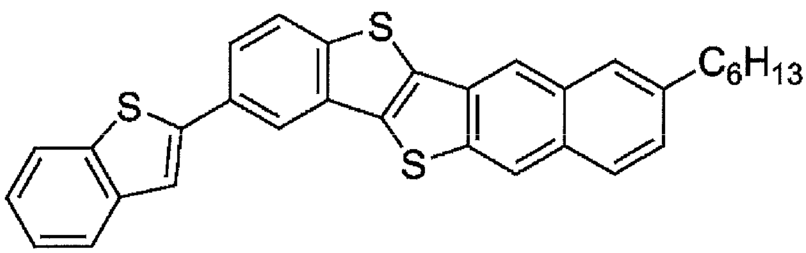

No.19

The measurement result of nuclear magnetic resonance spectroscopy for Organic compound 3 is given below.

$^1H$-NMR (400 MHz, $CDCl_3$) δ0.90 (t, 3H), 1.24-1.42 (m, 6H), 1.72 (Quin, 2H), 2.83 (t, 2H), 7.34-7.41 (m, 3H), 7.69 (s, 1H), 7.78-7.89 (m, 5H), 7.98 (d, 2H), 8.19 (d, 1H), 8.31 (s, 1H), 8.38 (s, 1H).

Example 4 (Synthesis of an organic compound according to the present invention, represented by No. 9 in the specific examples given above)

Except for using 2-[5-(1-butyl)-2-methylsulfinyl-1-phenyl]-6-phenylnaphtho[2,3-b]thiophene instead of 2-(2-methylsulfinyl-5-phenyl-1-phenyl)-6-hexylnaphtho[2,3-b]thiophene, the procedure of Example 1 was followed to give 1.2 g of Organic compound 4 according to the present invention. The yield of Organic compound 4 was 41%.

[Chemical Formula 13]

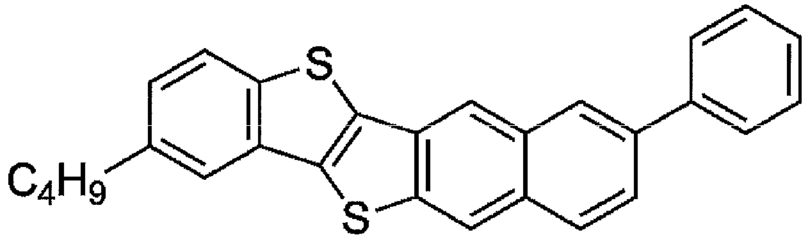

No.9

The measurement result of nuclear magnetic resonance spectroscopy for Organic compound 4 is given below. $^1H$-NMR (400 MHz, $CDCl_3$) δ0.98 (t, 3H), 1.38-1.48 (m, 2H), 1.68-1.76 (m, 2H), 2.80 (t, 2H), 7.29 (dd, 1H), 7.41 (t, 1H), 7.50-7.54 (m, 2H), 7.71 (s, 1H), 7.77-7.79 (m, 2H), 7.80 (dd, 1H), 7.85 (d, 1H), 8.01 (d, 1H), 8.22 (s, 1H), 8.40 (s, 1H), 8.41 (s, 1H).

Example 5 (Synthesis of an organic compound according to the present invention, represented by No. 11 in the specific examples given above)

Except for using 2-[2-methylsulfinyl-5-(1-octyl)-1-phenyl]-6-phenylnaphtho[2,3-b]thiophene instead of 2-(2-methylsulfinyl-5-phenyl-1-phenyl)-6-hexylnaphtho[2,3-b]thiophene, the procedure of Example 1 was followed to give 1.1 g of Organic compound 5 according to the present invention. The yield of Organic compound 5 was 28%.

[Chemical Formula 14]

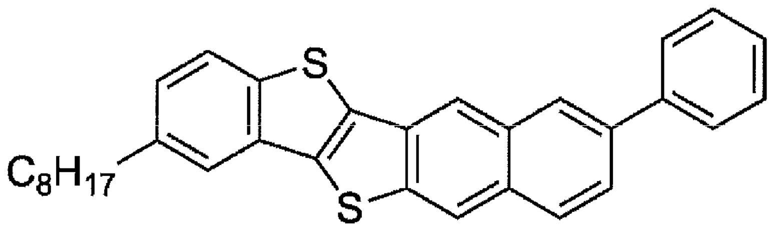

No.11

The measurement result of nuclear magnetic resonance spectroscopy for Organic compound 5 is given below. $^1H$-NMR (400 MHz, $CDCl_3$) δ0.89 (t, 3H), 1.28-1.44 (m, 10H), 1.69-1.77 (m, 2H), 2.80 (t, 2H), 7.29 (dd, 1H), 7.39-7.43 (m, 1H), 7.50-7.54 (m, 2H), 7.70 (s, 1H), 7.77-7.81 (m, 3H), 7.85 (d, 1H), 8.01 (d, 1H), 8.22 (s, 1H), 8.40 (s, 1H), 8.41 (s, 1H).

Example 6 (Synthesis of an organic compound according to the present invention, represented by No. 5 in the specific examples given above)

Except for using 2-(2-methylsulfinyl-5-phenyl-1-phenyl)-6-decylnaphtho[2,3-b]thiophene instead of 2-(2-methylsulfinyl-5-phenyl-1-phenyl)-6-hexylnaphtho[2,3-b]thiophene, the procedure of Example 1 was followed to give 2.5 g of Organic compound 6 according to the present invention. The yield of Organic compound 6 was 35%.

[Chemical Formula 15]

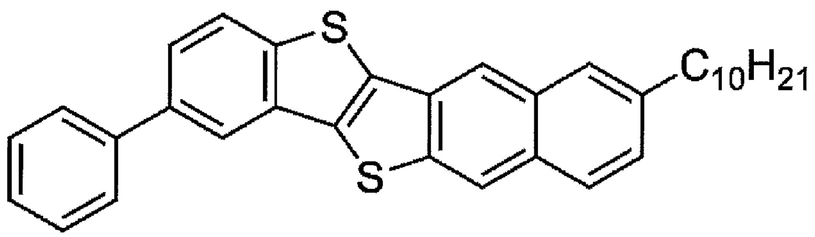

No.5

The measurement result of nuclear magnetic resonance spectroscopy for Organic compound 6 is given below. $^1$H-NMR (400 MHz, $CDCl_3$) δ0.87 (t, 3H), 1.26-1.42 (m, 14H), 1.69-1.77 (m, 2H), 2.81 (t, 2H), 7.35-7.41 (m, 2H), 7.48-7.52 (m, 2H), 7.66 (dd, 1H), 7.70-7.72 (m, 2H), 7.77 (s, 1H), 7.84 (d, 1H), 8.00 (d, 1H), 8.06 (s, 1H), 8.29 (s, 1H), 8.35 (s, 1H).

Example 7 (Synthesis of an organic compound according to the present invention, represented by No. 27 in the specific examples given above)

Except for using 6-hexyl-2-[5-(4-methylphenyl)-2-methylsulfinyl-1-phenyl]naphtho[2,3-b]thiophene instead of 2-(2-methylsulfinyl-5-phenyl-1-phenyl)-6-hexylnaphtho[2,3-b]thiophene, the procedure of Example 1 was followed to give 1.2 g of Organic compound 7 according to the present invention. The yield of Organic compound 7 was 12%.

[Chemical Formula 16]

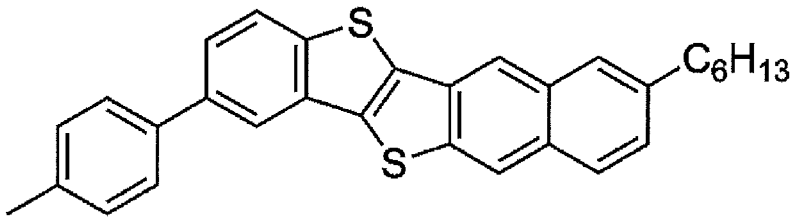

No.27

The measurement result of nuclear magnetic resonance spectroscopy for Organic compound 7 is given below. $^1$H-NMR (400 MHz, $CDCl_3$) δ0.89 (t, 3H), 1.24-1.40 (m, 6H), 1.69-1.77 (m, 2H), 2.43 (s, 3H), 2.81 (t, 2H), 7.30 (d, 2H), 7.36 (dd, 1H), 7.61 (d, 2H), 7.64 (dd, 1H), 7.77 (s, 1H), 7.84 (d, 1H), 7.97 (d, 1H), 8.04 (d, 1H), 8.29 (s, 1H), 8.34 (s, 1H).

Example 8 (Synthesis of an organic compound according to the present invention, represented by No. 29 in the specific examples given above)

Except for using 2-[5-(4-ethylphenyl)-2-methylsulfinyl-1-phenyl]-6-hexylnaphtho[2,3-b]thiophene instead of 2-(2-methylsulfinyl-5-phenyl-1-phenyl)-6-hexylnaphtho[2,3-b]thiophene, the procedure of Example 1 was followed to give 1.6 g of Organic compound 8 according to the present invention. The yield of Organic compound 8 was 14%.

[Chemical Formula 17]

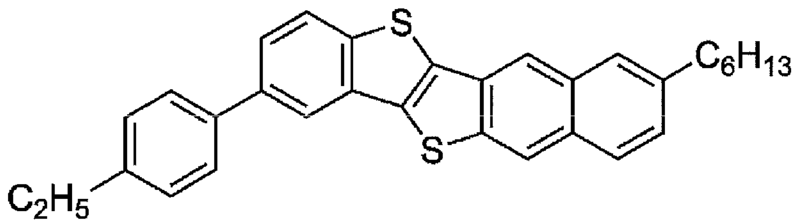

No.29

The measurement result of nuclear magnetic resonance spectroscopy for Organic compound 8 is given below. $^1$H-NMR (400 MHz, $CDCl_3$) δ0.88 (t, 3H), 1.29-1.42 (m, 9H), 1.68-1.77 (m, 2H), 2.72 (q, 2H), 2.81 (t, 2H), 7.32-7.34 (m, 2H), 7.37 (dd, 1H), 7.62-7.67 (m, 3H), 7.77 (s, 1H), 7.84 (d, 1H), 7.97 (d, 1H), 8.05 (d, 1H), 8.29 (s, 1H), 8.35 (s, 1H).

Example 9 (Synthesis of an organic compound according to the present invention, represented by No. 34 in the specific examples given above)

Except for using 2-[2-methylsulfinyl-5-(4-pentylphenyl)-1-phenyl]-6-phenylnaphtho[2,3-b]thiophene instead of 2-(2-methylsulfinyl-5-phenyl-1-phenyl)-6-hexylnaphtho[2,3-b]thiophene, the procedure of Example 1 was followed to give 0.8 g of Organic compound 9 according to the present invention. The yield of Organic compound 9 was 28%.

[Chemical Formula 18]

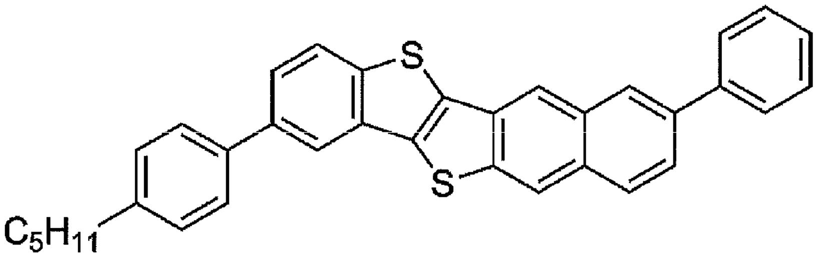

No.34

The measurement result of nuclear magnetic resonance spectroscopy for Organic compound 9 is given below. $^1$H-NMR (400 MHz, $CDCl_3$) δ0.93 (t, 3H), 1.36-1.41 (m, 4H), 1.65-1.73 (m, 2H), 2.69 (t, 2H), 7.32 (d, 2H), 7.40-7.43 (m, 1H), 7.51-7.55 (m, 2H), 7.65 (d, 2H), 7.68 (dd, 1H), 7.77-7.83 (m, 3H), 8.00 (d, 1H), 8.02 (d, 1H), 8.08 (d, 1H), 8.24 (d, 1H), 8.43 (s, 2H).

Comparative examples 1-3

An organic compound in Comparative example 1 is represented by No. 35 below (a compound similar to the one disclosed in PTL 4). An organic compound in Comparative example 2 is represented by No. 36 below (a compound similar to Organic compound 1 but having a different substitution position). An organic compound in Comparative example 3 is represented by No. 37 below (a compound disclosed in NPL 4).

[Chemical Formula 19]

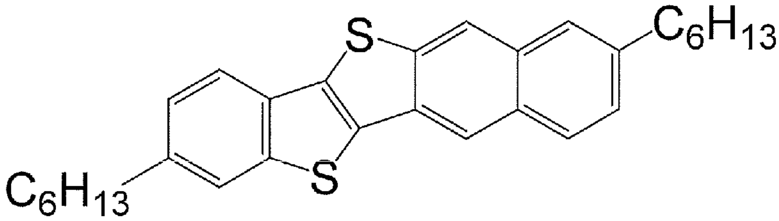

No.35

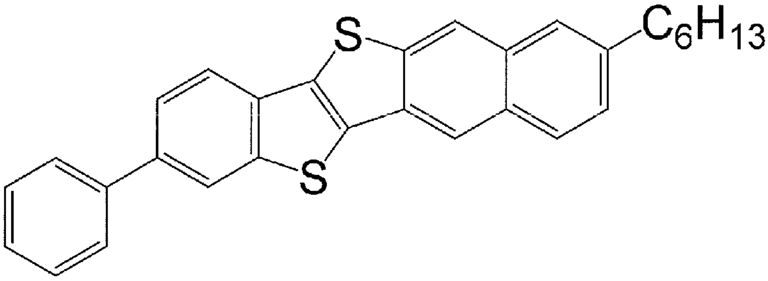

No.36

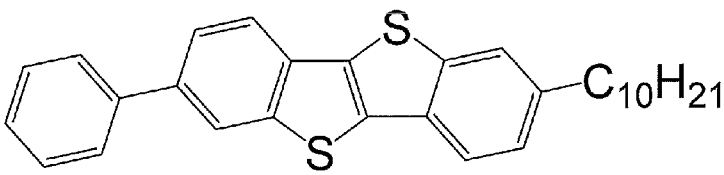

No.37

Example 10 (Differential scanning calorimetry)

Differential scanning calorimetry was performed for Organic compounds 1-6, 8, and 9 obtained in Examples 1-6, 8, and 9, and for organic compounds in Comparative examples 1-3 in the following manner.

About 2 mg of each organic compound powder was weighed out and fed hermetically in an aluminum pan. Each sample was set on a differential scanning calorimeter and heated up to 550°C. During this heating, the endothermic peak was observed to find a transition point (a peak top temperature; a peak indicating the phase transition). The results are given in Table 1.

[Table 1]

| Organic compound | Peak top temperature (°C) |
|---|---|
| 1 | 201 |
| 2 | 193 |
| 3 | 260 |
| 4 | 218 |
| 5 | 186 |
| 6 | 185 |
| 8 | 229 |
| 9 | 244 |
| Comp. Ex. 1 | 182 |
| Comp. Ex. 2 | 281 |
| Comp. Ex. 3 | 143 |

The organic compound in Comparative example 3 (the compound disclosed in NPL 4) having four fused aromatic rings showed an endothermic peak (a peak indicating phase transition) at not higher than 150°C, and thus did not have satisfactory heat resistance. On the other hand, Organic compounds 1-6, 8, and 9 obtained in Examples 1-6, 8, and 9 of the present invention and each having five fused aromatic ring compounds showed endothermic peaks at 150°C or higher (to be more specific, their endothermic peaks were higher by 40°C or more, compared with the organic compound in Comparative example 3), and thus proved their high heat resistance.

Example 11 (Measurement of solubility)

For Organic compounds 1-6 and 8 obtained in Examples 1-6 and 8, and the organic compounds obtained in Comparative examples 1-3, solubility was measured by the following method.

About 1 mg of each organic compound powder was weighed and fed into vials, and the weight of the content in each vial was measured precisely. Into these vials, chloroform, toluene, and tetrahydronaphthalene (THN) were added at 25°C (room temperature). When the added solvent dissolved completely, the mass was measured precisely again. The mass of the organic compound was divided by the total weight of the organic compound and the added solvent, and was multiplied by 100 to obtain solubility. Dissolution or non-dissolution was judged by visual inspection. The results are shown in Table 2.

[Table 2]

| Organic Compound | Solubility in organic solvent at 25°C (mass%) | | | Solution state after one week |
|---|---|---|---|---|
| | chloroform | toluene | THN | |
| 1 | 1.3 | 0.4 | 0.7 | no precipitation |
| 2 | 0.5 | 0.3 | 0.4 | no precipitation |
| 3 | 0.5 | 0.3 | 0.3 | no precipitation |
| 4 | 0.5 | 0.5 | 0.5 | no precipitation |
| 5 | 0.4 | 0.4 | 0.4 | no precipitation |

(continued)

| Organic Compound | Solubility in organic solvent at 25°C (mass%) | | | Solution state after one week |
|---|---|---|---|---|
| | chloroform | toluene | THN | |
| 6 | 0.3 | 0.2 | 0.3 | no precipitation |
| 8 | >2 | 0.9 | 1.4 | no precipitation |
| Comp. Ex. 1 | 0.5 | 0.5 | 0.5 | no precipitation |
| Comp. Ex. 2 | <0.1 | <0.1 | <0.1 | precipitation |
| Comp. Ex. 3 | 0.3 | 0.2 | 0.2 | no precipitation |

The difference between Organic compound 1 obtained in Example 1 of the present invention and the organic compound in Comparative example 2 resides merely in the substitution position of the substituent in the BTNT skeleton. Owing to this slight difference, as evident from Table 2, the solubility of the former in the organic solvent at 25°C was 10 times or more higher, at the maximum, than that of the latter. Generally speaking, an increase in the number of alkyl chains improves solubility in the organic solvent, and introduction of a phenyl group deteriorates solubility in the organic solvent. It is therefore surprising that Organic compound 1 having one alkyl chain and one phenyl group showed higher solubility than the organic compound in Comparative example 1 having two alkyl chains. According to Table 2, it is also understood that Organic compounds 2-6 and 8 obtained in Examples 2-6 and 8 of the present invention showed notably high solubility in the organic solvent at 25°C than the organic compound in Comparative example 2 in which the substitution position of the substituent in the BTNT skeleton was different. From these results, it is found that the substituent introduction position in the BTNT skeleton of the organic compounds according to the present invention plays a very effective part in improving solubility.

From Table 2, it is also found that Organic compounds 1-6 and 8 obtained in Examples 1-6 and 8 of the present invention showed higher storage stability in a solution state than the organic compound in Comparative example 2 in which the substitution position of the substituent in the BTNT skeleton was different.

Example 6 (Evaluation of organic transistor characteristics)

Organic transistors were manufactured with use of Organic compounds 1, 2, 5, and 6 obtained in Examples 1, 2, 5, and 6, and the organic compounds in Comparative examples 1 and 2. Characteristics of the obtained transistors were evaluated.

Using the solutions containing Organic compounds 1, 2, 5, and 6 which were obtained in Example 11, organic thin films were formed on n-doped silicon wafers each having a $SiO_2$ thermal oxide film, at 25°C by spin coating. On each of the organic thin films, Au was vacuum deposited using a shadow mask to give a source electrode and a drain electrode. For these organic transistors, the channel length was set at 50 $\mu$m, and the channel width was set at 2.5 mm. The thus produced organic transistor had a top-contact bottom-gate structure, as shown in Fig. 1(b). In the organic transistors of these Examples, each being composed of the n-doped silicon wafer having a $SiO_2$ thermal oxide film, the $SiO_2$ thermal oxide film served as the insulator layer (4), and the n-doped silicon wafer served as both the substrate (6) and the gate electrode (5).

The performance of the organic transistor depends on the amount of electric current that flows when an electrical potential is applied between the source electrode and the drain electrode while an electrical potential is kept applied to the gate electrode. Mobility, which is one of the characteristics of the organic transistor, can be determined by measuring the value of the electric current. Mobility can be calculated by Formula (a) which expresses electrical characteristics of carrier seeds generated in the organic semiconductor layer when a gate electrical field is applied to the $SiO_2$ thermal oxide film as the insulator layer.

$$Id = Z\mu Ci(Vg\text{-}Vt)^2/2L \ldots \text{(a)}$$

In this formula, Id represents the saturated source-drain current value, Z represents the channel width, Ci represents the capacitance of the insulator, Vg represents the gate electrical potential, Vt represents the threshold potential, L represents the channel length, and $\mu$ represents the mobility to be determined ($cm^2/Vs$). Ci is determined by the dielectric constant of the $SiO_2$ insulating film. Z and L are determined by the device structure of the organic transistor. Id and Vg are determined when the value of the electric current of the organic transistor is measured. Vt is obtained from Id and Vg. By assigning these values to Formula (a), mobility at the gate electrical potential of each organic transistor can be

calculated.

Fig. 3 shows transfer curves that indicate changes in drain current in the thus produced organic transistors, at the drain voltage of -30V and the gate voltage swept from +20V to -80V. The hole mobility, the threshold voltage, and the ratio of ON-current to OFF-current ("on/off ratio") of the organic transistor, calculated from Formula (a), are shown in Table 3.

[Table 3]

| Organic Compounds | Hole mobility ($cm^2/Vs$) | Threshold voltage (V) | On/off ratio |
|---|---|---|---|
| 1 | 1.62 | -28 | $10^9$ |
| 2 | 0.62 | -2.3 | $10^8$ |
| 5 | 2.58 | -4.8 | $10^8$ |
| 6 | 1.97 | -23 | $10^7$ |
| Comp. Ex. 1 | $1.19\times10^{-2}$ | -11 | $10^3$ |
| Comp. Ex. 2 | Film not formed due to poor solubility | | |

According to Table 3, Organic compounds 1, 2, 5, and 6 obtained in Examples 1, 2, 5, and 6 of the present invention were applicable to the solution process, whereas the organic compound of Comparative example 2 was not. The mobilities of the organic transistors produced by the solution process was 50 to 250 times higher than the mobility of the transistor produced with use of the organic compound in Comparative example 1 in which two alkyl groups were introduced in the BTNT skeleton. It is therefore evident that Organic compounds obtained in Examples 1, 2, 5, and 6 of the present invention showed higher mobilities and greater on/off ratios.

From these findings, the organic compounds according to the present invention are found to be excellent organic semiconductor materials, characterized by: having better heat resistance than the organic compound having four fused aromatic rings; having better solubility in the organic solvent at room temperature and better storage stability in a solution state than the organic compound in which the substitution position of the substituent in the BTNT skeleton was different; and being capable of providing organic transistors with higher mobilities and greater on/off ratios than the organic compound in which two alkyl groups were introduced in the BTNT skeleton.

Industrial Applicability

Regarding the organic compounds (condensed polycyclic aromatic compounds) and organic semiconductor materials, the present invention can improve solubility in an organic solvent at room temperature, storage stability in a solution state, heat resistance, and hole mobility, as described above. Therefore, it is possible to provide organic compounds (condensed polycyclic aromatic compounds) and organic semiconductor materials which are highly heat-resistant and to which a solution process is applicable. As a result, the present invention is applicable to the fields such as organic transistor devices, diodes, capacitors, thin-film photoelectric conversion devices, dye-sensitized solar cells, and organic EL devices.

Reference Signs List

In Figs. 1 and 2, the same components are indicated by the same reference numerals.

1 source electrode
2 semiconductor layer
3 drain electrode
4 insulator layer
5 gate electrode
6 substrate
7 protective layer
10A-10F organic transistor

The present invention can be embodied and practiced in other different forms without departing from the present invention. Therefore, the above-described embodiments are considered in all respects as illustrative and not restrictive. The scope of the invention is indicated by the appended claims rather than by the foregoing description.

**Claims**

1. An organic compound represented by Formula (A),

[Chemical Formula 1]

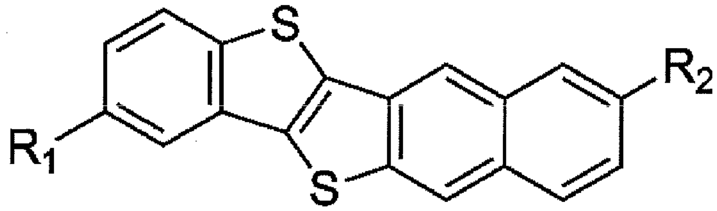

(A)

wherein one of $R_1$ and $R_2$ represents an alkyl group, an aromatic hydrocarbon group having an alkyl group, or a heterocyclic group having an alkyl group, and another of $R_1$ and $R_2$ represents an aliphatic hydrocarbon group, an aromatic hydrocarbon group, or a heterocyclic group, with a proviso that $R_1$ and $R_2$ are not alkyl groups simultaneously.

2. The organic compound according to claim 1,
wherein one of $R_1$ and $R_2$ is an aromatic hydrocarbon group which has an alkyl group having 2 to 16 carbon atoms, or a heterocyclic group which has an alkyl group having 2 to 16 carbon atoms.

3. The organic compound according to claim 2,
wherein one of $R_1$ and $R_2$ is an aromatic hydrocarbon group which has an alkyl group having 4 to 12 carbon atoms or a heterocyclic group which has an alkyl group having 4 to 12 carbon atoms, and another of $R_1$ and $R_2$ is an aromatic hydrocarbon group or a heterocyclic group.

4. The organic compound according to claim 1,
wherein one of $R_1$ and $R_2$ is a straight-chain alkyl group having 4 to 10 carbon atoms, and another of $R_1$ and $R_2$ is an aromatic hydrocarbon group or a heterocyclic group.

5. The organic compound according to claim 1,
wherein one of $R_1$ and $R_2$ is a branched-chain alkyl group having 6 to 12 carbon atoms, and another of $R_1$ and $R_2$ is an aromatic hydrocarbon group or a heterocyclic group.

6. The organic compound according to any one of claims 1 to 5,
wherein no peak indicating a phase transition is observed below 150°C in differential scanning calorimetry of the organic compound.

7. The organic compound according to any one of claims 1 to 6, having solubility in an organic solvent at 25°C is 0.1 mass% or higher.

8. The organic compound according to claim 7, having solubility in an organic solvent at 25°C is 0.3 mass% or higher.

9. The organic compound according to claim 7 or 8,
wherein the organic solvent is a non-halogenated organic solvent.

10. An organic semiconductor material which comprises the organic compound according to any one of claims 1 to 9.

11. An organic thin film which comprises the organic semiconductor material according to claim 10.

12. An organic semiconductor composition which comprises the organic compound according to any one of claims 1 to 9 and an organic solvent.

13. The organic semiconductor composition according to claim 12,
wherein a content of the organic compound is 0.1 to 5 mass%.

**14.** A method for producing an organic thin film which comprises the steps of:

applying or printing the organic semiconductor composition according to claim 12 or 13; and
drying the composition.

**15.** An organic semiconductor device which comprises the organic thin film according to claim 11.

**16.** The organic semiconductor device according to claim 15, wherein the device is an organic transistor.

**Patentansprüche**

**1.** Eine organische Verbindung, die durch die Formel (A),

[Chemische Formel 1]

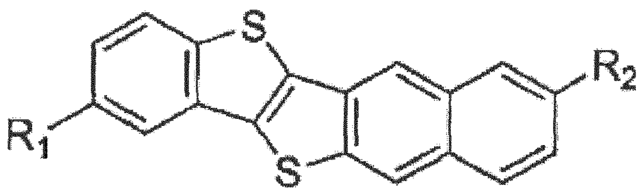

(A)

dargestellt wird,
worin eines von $R_1$ und $R_2$ eine Alkylgruppe, eine aromatische Kohlenwasserstoffgruppe, die eine Alkylgruppe aufweist, oder eine heterocyclische Gruppe, die eine Alkylgruppe aufweist, darstellt, und das andere von $R_1$ und $R_2$ eine aliphatische Kohlenwasserstoffgruppe, eine aromatische Kohlenwasserstoffgruppe, oder eine heterocyclische Gruppe darstellt, mit der Maßgabe, dass $R_1$ und $R_2$ nicht gleichzeitig Alkylgruppen sind.

**2.** Die organische Verbindung gemäß Anspruch 1,
worin eines von $R_1$ und $R_2$ eine aromatische Kohlenwasserstoffgruppe, die eine Alkylgruppe mit 2 bis 16 Kohlenstoffatomen aufweist, oder eine heterocyclische Gruppe, die eine Alkylgruppe mit 2 bis 16 Kohlenstoffatomen aufweist, ist.

**3.** Die organische Verbindung gemäß Anspruch 2,
worin eines von $R_1$ und $R_2$ eine aromatische Kohlenwasserstoffgruppe, die eine Alkylgruppe mit 4 bis 12 Kohlenstoffatomen aufweist, oder eine heterocyclische Gruppe, die eine Alkylgruppe mit 4 bis 12 Kohlenstoffatomen aufweist, ist, und das andere von $R_1$ und $R_2$ eine aromatische Kohlenwasserstoffgruppe oder eine heterocyclische Gruppe ist.

**4.** Die organische Verbindung gemäß Anspruch 1,
worin eines von $R_1$ und $R_2$ eine geradkettige Alkylgruppe mit 4 bis 10 Kohlenstoffatomen, und das andere von $R_1$ und $R_2$ eine aromatische Kohlenwasserstoffgruppe oder eine heterocyclische Gruppe ist.

**5.** Die organische Verbindung gemäß Anspruch 1,
worin eines von $R_1$ und $R_2$ eine verzweigtkettige Alkylgruppe mit 6 bis 12 Kohlenstoffatomen ist, und das andere von $R_1$ und $R_2$ eine aromatische Kohlenwasserstoffgruppe oder eine heterocyclische Gruppe ist.

**6.** Die organische Verbindung gemäß einem der Ansprüche 1 bis 5,
wobei bei der dynamischen Differemzkalorimetrie der organischen Verbindung unterhalb von 150 °C kein Peak beobachtet wird, der einen Phasenübergang anzeigt.

**7.** Die organische Verbindung gemäß einem der Ansprüche 1 bis 6,
die eine Löslichkeit in einem organischen Lösungsmittel bei 25 °C von 0,1 Gewichts-% oder höher aufweist.

**8.** Die organische Verbindung gemäß Anspruch 7,
die eine Löslichkeit in einem organischen Lösungsmittel bei 25 °C von 0,3 Gewichts-% oder höher aufweist.

9. Die organische Verbindung gemäß Anspruch 7 oder 8,
wobei das organische Lösungsmittel ein nicht-halogeniertes organisches Lösungsmittel ist.

10. Ein organisches Halbleitermaterial, das die organische Verbindung gemäß einem der Ansprüche 1 bis 9 umfasst.

11. Eine organische Dünnschicht, die das organische Halbleitermaterial gemäß Anspruch 10 umfasst.

12. Eine organisches Halbleiterzusammensetzung, die eine organische Verbindung gemäß einem der Ansprüche 1 bis 9 und ein organisches Lösungsmittel umfasst.

13. Die organische Halbleiterzusammensetzung gemäß Anspruch 12,
wobei der Gehalt der organischen Verbindung 0,1 bis 5 Gewichts-% beträgt.

14. Ein Verfahren zur Herstellung einer organischen Dünnschicht, das die Schritte umfasst:

Das Auftragen oder Drucken der organischen Halbleiterzusammensetzung gemäß Anspruch 12 oder 13; und das Trocknen der Zusammensetzung.

15. Eine organische Halbleitervorrichtung, die die organische Dünnschicht gemäß Anspruch 11 umfasst.

16. Die organische Halbleitervorrichtung gemäß Anspruch 15, wobei die Vorrichtung ein organischer Transistor ist.

**Revendications**

1. Composé organique représenté par la formule (A),

[Formule chimique 1]

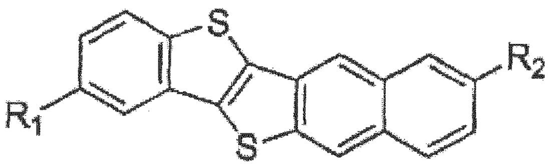

(A)

dans laquelle l'un de $R_1$ et $R_2$ représente un groupe alkyle, un groupe hydrocarboné aromatique ayant un groupe alkyle, ou un groupe hétérocyclique ayant un groupe alkyle, et l'autre de $R_1$ et $R_2$ représente un groupe hydrocarboné aliphatique, un groupe hydrocarboné aromatique, ou un groupe hétérocyclique, à condition que $R_1$ et $R_2$ ne soient pas simultanément des groupes alkyle.

2. Composé organique selon la revendication 1,
dans lequel l'un de $R_1$ et $R_2$ est un groupe hydrocarboné aromatique qui a un groupe alkyle ayant 2 à 16 atomes de carbone, ou un groupe hétérocyclique qui a un groupe alkyle ayant de 2 à 16 atomes de carbone.

3. Composé organique selon la revendication 2,
dans lequel l'un de $R_1$ et $R_2$ est un groupe hydrocarboné aromatique qui a un groupe alkyle ayant 4 à 12 atomes de carbone ou un groupe hétérocyclique qui a un groupe alkyle ayant 4 à 12 atomes de carbone, et l'autre de $R_1$ et $R_2$ est un groupe hydrocarboné aromatique ou un groupe hétérocyclique.

4. Composé organique selon la revendication 1,
dans lequel l'un de $R_1$ et $R_2$ est un groupe alkyle à chaîne droite ayant 4 à 10 atomes de carbone, et l'autre de $R_1$ et $R_2$ est un groupe hydrocarboné aromatique ou un groupe hétérocyclique.

5. Composé organique selon la revendication 1,
dans lequel l'un de $R_1$ et $R_2$ est un groupe alkyle à chaîne ramifiée ayant 6 à 12 atomes de carbone, et l'autre de $R_1$ et $R_2$ est un groupe hydrocarboné aromatique ou un groupe hétérocyclique.

6. Composé organique selon l'une quelconque des revendications 1 à 5,
dans lequel aucun pic indiquant une transition de phase n'est observé en dessous de 150°C lors d'une calorimétrie à balayage différentiel du composé organique.

7. Composé organique selon l'une quelconque des revendications 1 à 6, ayant une solubilité dans un solvant organique à 25°C supérieure ou égale à 0,1% en masse.

8. Composé organique selon la revendication 7, ayant une solubilité dans un solvant organique à 25°C supérieure ou égale à 0,3% en masse.

9. Composé organique selon la revendication 7 ou 8,
dans lequel le solvant organique est un solvant organique non halogéné.

10. Matériau semi-conducteur organique qui comprend le composé organique selon l'une quelconque des revendications 1 à 9.

11. Film mince organique qui comprend le matériau semi-conducteur organique selon la revendication 10.

12. Composition semi-conductrice organique qui comprend le composé organique selon l'une quelconque des revendications 1 à 9 et un solvant organique.

13. Composition semi-conductrice organique selon la revendication 12, dans laquelle la teneur en composé organique est de 0,1 à 5% en masse.

14. Procédé de production d'un film mince organique qui comprend les étapes consistant :

à appliquer ou imprimer la composition semi-conductrice organique selon la revendication 12 ou 13 ; et
à sécher la composition.

15. Dispositif à semi-conducteur organique qui comprend le film mince organique selon la revendication 11.

16. Dispositif à semi-conducteur organique selon la revendication 15, dans lequel le dispositif est un transistor organique.

FIG. 1(a)

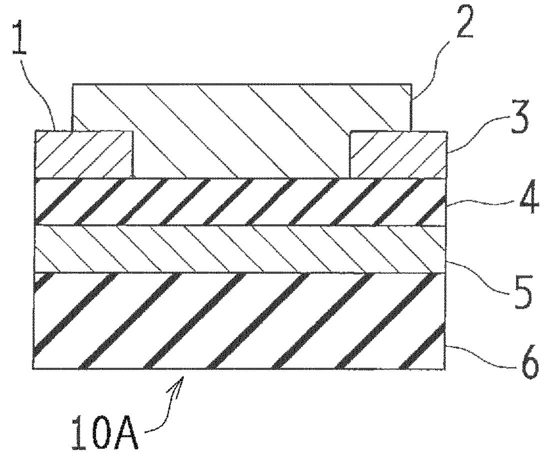


FIG. 1(b)

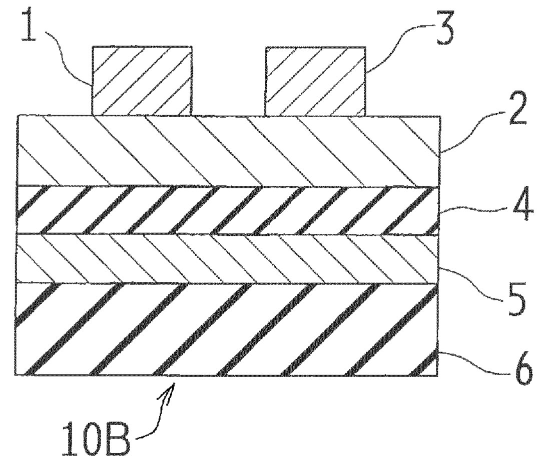


FIG. 1(c)

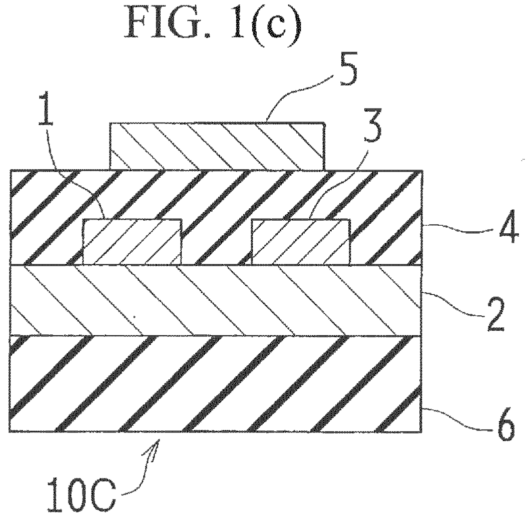


FIG. 1(d)

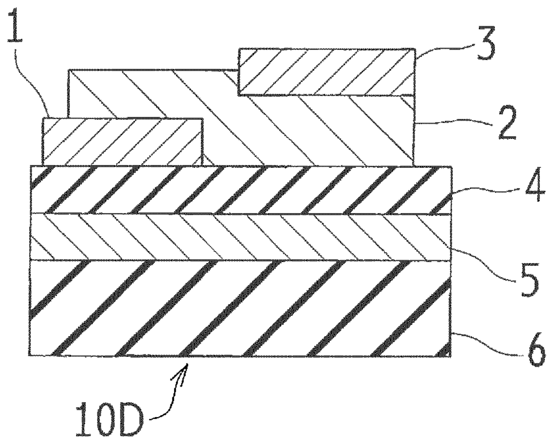


FIG. 1(e)

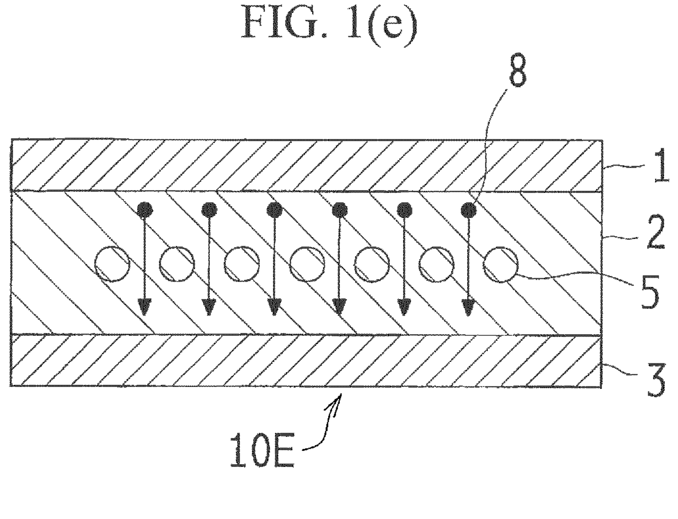


FIG. 1(f)

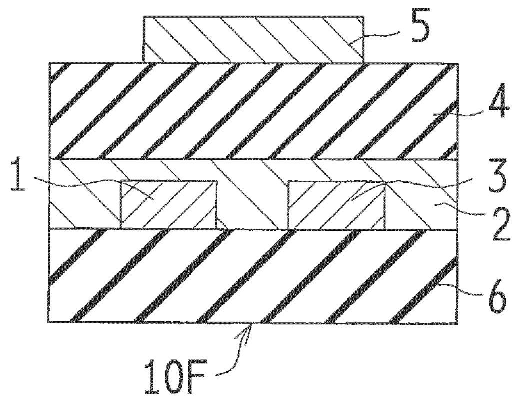

FIG. 2

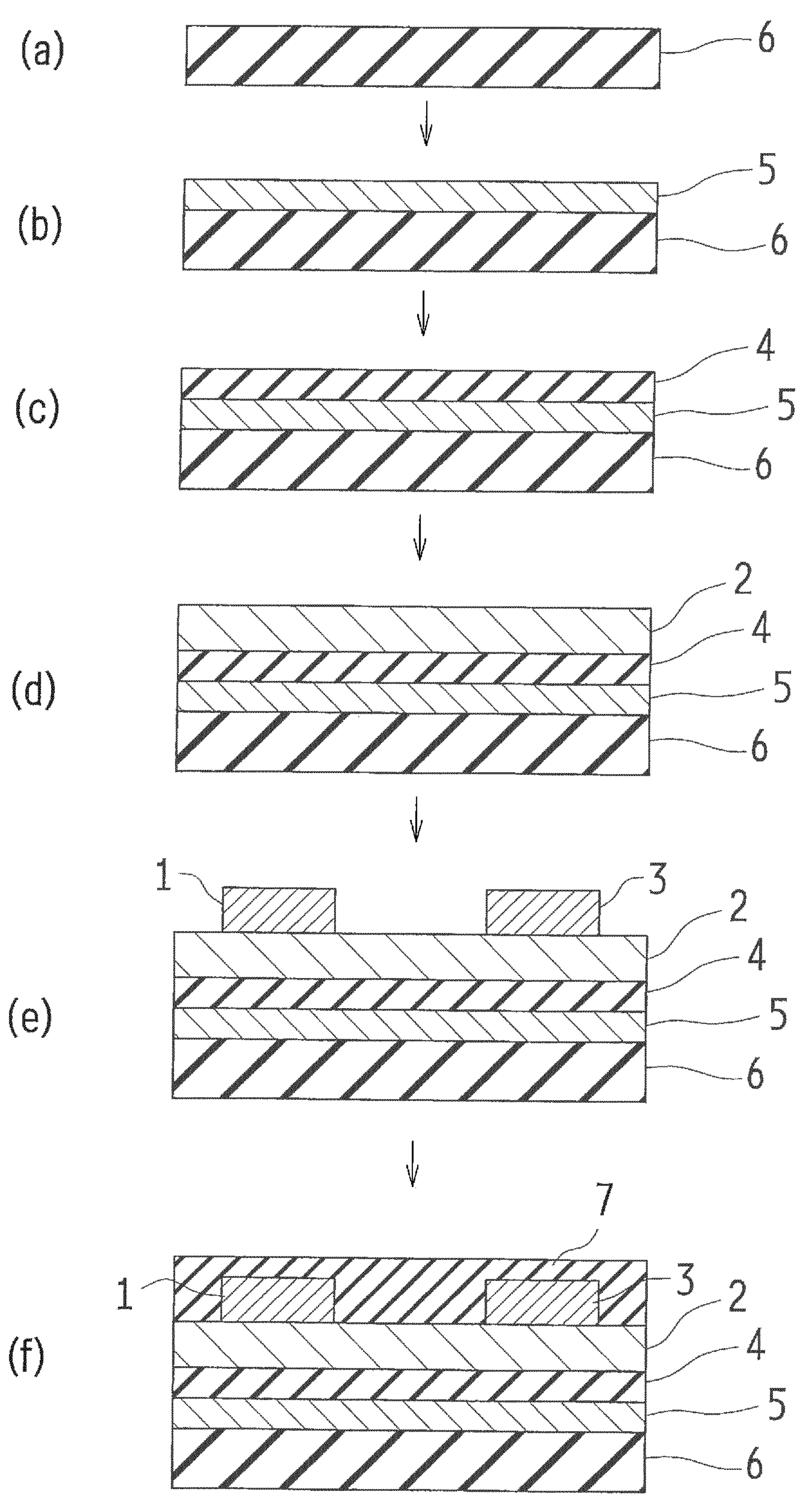

FIG. 3

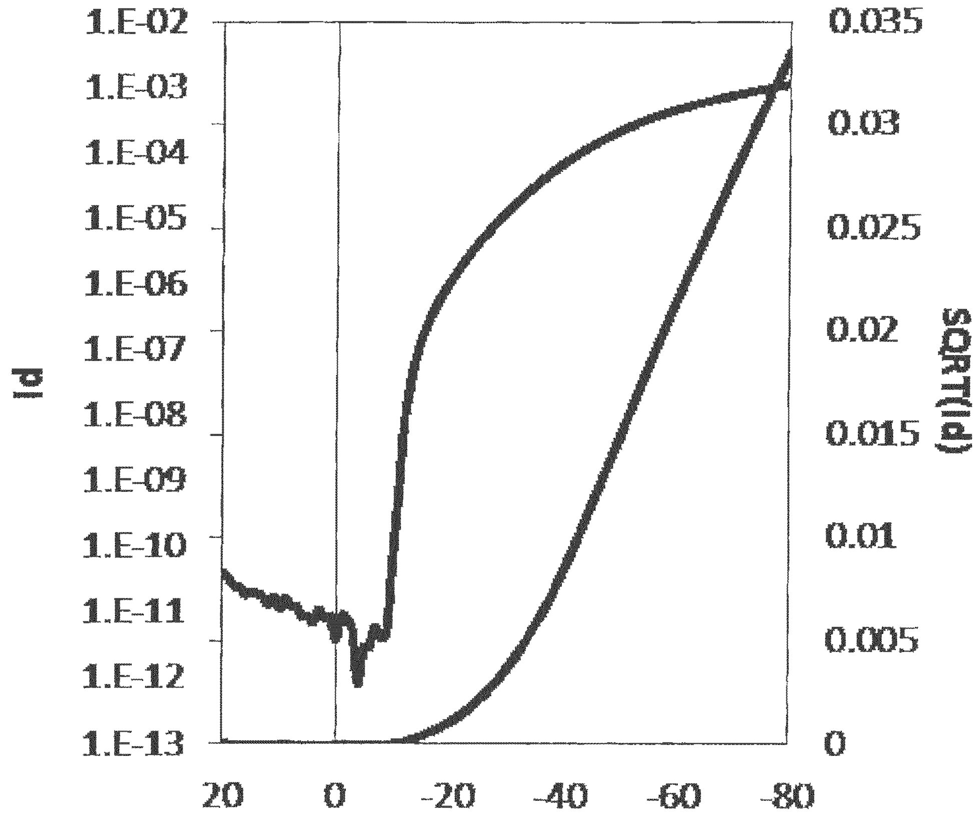
1.E-02
1.E-03
1.E-04
1.E-05
1.E-06
1.E-07
1.E-08
1.E-09
1.E-10
1.E-11
1.E-12
1.E-13
Id
0.035
0.03
0.025
0.02
0.015
0.01
0.005
0
SQRT(Id)
20
0
-20
-40
-60
-80

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008258592 A
- WO 2008047896 A1
- WO 2010098372 A1
- JP 2010034450 A
- JP 2014531435 A
- JP 2011256144 A


### Non-patent literature cited in the description


- *Chemistry Letters,* 2008, vol. 37 (3), 284-285
- *Journal of the American Chemical Society,* 2007, vol. 129 (51), 15732-15733
- *Advanced Materials,* 2011, vol. 23 (10), 1222-1225
- *Nature Communications,* 10 April 2015, vol. 6